# FASCICULE DE BREVET EUROPEEN

(11) **EP 3 583 424 B1**
(45) Date de publication et mention de la délivrance du brevet: **11.05.2022**
(21) Numéro de dépôt: 18709063.4
(22) Date de dépôt: 16.02.2018
(51) Int. Cl.: G01N 33/569

(54) **METHODE DE DOSAGE IMMUNOLOGIQUE D'ANTICORPS SPECIFIQUES D'ANTIGENES DE VIRUS DE LA FAMILLE DESPOXVIRIDAE**
VERFAHREN ZUR IMMUNOLOGISCHEN DOSIERUNG SPEZIFISCHER ANTIKÖRPER VON ANTIGENEN VON VIREN VON THEPOXVIRIDAE
METHOD FOR THE IMMUNOLOGICAL DOSING OF SPECIFIC ANTIBODIES OF ANTIGENS OF VIRUSES OF THEPOXVIRIDAE

(30) Priorité: 16.02.2017 FR 1751266
(43) Date de publication de la demande: 25.12.2019
(73) Titulaire: Innovative Diagnostics, 34790 Grabels (FR)
(72) Inventeur: POURQUIER, Philippe, 34070 Montpellier (FR); COMTET, Loïc, 34790 Grabels (FR); CARPENTIER, Alix, 34130 Candillargues (FR); ROCHE, Mickaël, 34090 Montpellier (FR)
(74) Mandataire: Regimbeau
(86) Numéro de dépôt international: PCT/FR2018/050376
(87) Numéro de publication internationale: WO 2018/150148

(56) Documents cités:
- WO-A2-2009/048769
- CN-A- 102 981 000
- TIAN HONG ET AL: "Serodiagnosis of sheeppox and goatpox using an indirect ELISA based on synthetic peptide targeting for the major antigen P32", VIROLOGY JOURNAL, BIOMED CENTRAL, LONDON, GB, vol. 7, no. 1, 21 septembre 2010 (2010-09-21), page 245, XP021080175, ISSN: 1743-422X, DOI: 10.1186/1743-422X-7-245
- PINGFAN YUAN ET AL: "Multicolor Quantum Dot-Encoded Microspheres for the Fluoroimmunoassays of Chicken Newcastle Disease and Goat Pox Virus", JOURNAL OF NANOSCIENCE AND NANOTECHNOLOGY, vol. 9, no. 5, 2 mai 2009 (2009-05-02), pages 3092-3098, XP055404651, US ISSN: 1533-4880, DOI: 10.1166/jnn.2009.009
- BOWDEN T R ET AL: "Detection of antibodies specific for sheeppox and goatpox viruses using recombinant capripoxvirus antigens in an indirect enzyme-linked immunosorbent assay", JOURNAL OF VIROLOGICAL METHODS, ELSEVIER BV, NL, vol. 161, no. 1, 1 octobre 2009 (2009-10-01), pages 19-29, XP026349457, ISSN: 0166-0934 [extrait le 2009-05-06]
- CARN V M ET AL: "Use of a recombinant antigen in an indirect ELISA for detecting bovine antibody to capripoxvirus", JOURNAL OF VIROLOGICAL METHODS, ELSEVIER BV, NL, vol. 49, no. 3, 1 octobre 1994 (1994-10-01), pages 285-294, XP025440949, ISSN: 0166-0934, DOI: 10.1016/0166-0934(94)90143-0 [extrait le 1994-10-01]
- Kristine Klewer-Fromentin ET AL: "Lumpy Skin Disease Validierung eines neuen ELISAs (und einer neuen qPCR Methode) für die Diagnostik der Lumpy Skin Krankheit", , 10 mai 2017 (2017-05-10), XP055404409, Extrait de l'Internet: URL:https://verbraucherschutz.sachsen-anha lt.de/fileadmin/Bibliothek/Politik_und_Ver waltung/MS/LAV_Verbraucherschutz/veterinae rmedizin/veranstaltungen/symposium_fb4/zeh ntes/08_Validierung_ELISAs_Real-Time_PCR__ Lumpy_Skin_Disease_Virus.pdf [extrait le 2017-09-06]

## Description

### DOMAINE DE L'INVENTION

La description porte sur une méthode de dosage immunologique à double antigène pour la détection d'anticorps d'un virus de la famille des *Poxviridae* dans un échantillon biologique, ainsi que le kit correspondant et ses utilisations, notamment pour la détection d'une infection ou le suivi d'une vaccination sur des animaux.

### BACKGROUND DE L'INVENTION

Les virus de la famille des *Poxviridae,* en particulier de la sous-famille des Chordopoxvirinae, sont répartis selon leurs genres et sont responsables de diverses maladies. On s'intéressera en particulier aux Orthopoxvirus, Parapoxvirus et Capripxovirus, et de préférence aux Capripoxvirus, sans que cela soit limitatif.

Le genre Orthopoxvirus comprend notamment le Cowpoxvirus (responsable de la variole bovine) et le Camelpoxvirus (responsable de la variole des camélidés), tous deux zoonotiques et présentant donc un risque de transmission à l'homme. Les bovins constituent le principal réservoir du virus cowpox. L'homme se contamine principalement par contact cutané avec un animal contaminé, même sans morsure ou griffure apparente. Cette zoonose se rencontre principalement chez les professionnels en contact avec des chats, des rongeurs ou des bovins : vétérinaires, éleveurs, personnels d'animaleries. Le virus de la variole des camélidés (camelpox) cause des maladies virales touchant principalement le chameau et le dromadaire, et cause des pertes économiques importantes en Afrique, au Moyen-Orient et en Asie.

Les maladies liées aux virus de la famille des *Poxviridae* et en particulier ceux du genre Capripoxvirus, qui se transmettent essentiellement par des piqures d'insectes et affectent le bétail, constituent un problème important de santé animale et préjudiciable sur le plan économique, car affectant les rendements de l'élevage du bétail.

La clavelée ou variole ovine (VO), la variole caprine (VC), ainsi que la dermatose nodulaire contagieuse pour les bovins (DNC ou LSD pour 'Lumpy Skin Disease') sont des maladies virales dues à un poxvirus ; elles évoluent soit sous une forme classique (vésiculeuse ou nodulaire), soit sous une forme compliquée, voire une forme suraiguë ou septicémique mais qui est rarement observée.

La dermatose nodulaire contagieuse (DNC) est généralement caractérisée par de la fièvre, l'apparition de volumineux nodules sur la peau, les muqueuses et les organes internes, de l'anémie, une hypertrophie des nœuds lymphatiques et de l'œdème cutané. Si la mortalité reste assez faible, la morbidité peut s'avérer importante et atteindre jusqu'à 90% dans une population indemne. Son importance économique est due aux pertes de production qu'elle occasionne, en particulier dans les troupeaux laitiers. Elle cause également des dommages importants aux cuirs. La dermatose nodulaire contagieuse (DNC) est causée par diverses souches de Capripoxvirus indistinctes au plan antigénique des souches responsables de la variole ovine (ou clavelée) et de la variole caprine. Cependant, la distribution géographique des deux affections est différente, ce qui suggère que les souches bovines de Capripoxvirus ne peuvent infecter ni se transmettre au mouton ou à la chèvre.

La dermatose nodulaire contagieuse (DNC), qui s'est étendue en Afrique depuis un siècle puis depuis 2012 au Moyen-Orient et vers le Sud-est européen, est maintenant émergente en Europe (cas repérés en 2015-2016 en Grèce, Bulgarie et d'autres pays des Balkans) et suscite un intérêt croissant pour trouver rapidement des solutions visant à prévenir et/ou lutter contre sa propagation.

Les experts mandatés par l'EFSA et la Commission européenne ont conclu dans un communiqué paru le 9 Août 2016, sur la base d'une étude de modélisation de l'efficacité relative de diverses mesures de lutte contre la propagation de cette maladie, que la vaccination était la meilleure méthode. Ainsi, si la vaccination est méticuleusement appliquée, l'abattage partiel des animaux atteints se révélerait aussi efficace pour éradiquer la maladie que l'abattage de troupeaux entiers, qui est actuellement exigé en vertu de la législation européenne. Et la vaccination est particulièrement efficace lorsqu'elle est appliquée avant que le virus ne s'introduise dans une région ou un pays.

Le dépistage sérologique d'animaux infectés est classiquement réalisé par l'épreuve de séroneutralisation virale, très spécifique. Cependant, l'immunité contre une infection à Capripoxvirus est surtout à médiation cellulaire. L'épreuve n'est donc pas suffisamment sensible pour identifier les animaux ayant été en contact avec le virus et qui n'ont élaboré que de faibles quantités d'anticorps neutralisants. Ces anticorps neutralisants n'expliquent pas à eux seuls l'immunité : une protection solide peut s'accompagner d'un faible taux d'anticorps neutralisants. La méthode du Western blot se basant sur la réaction entre un antigène spécifique du Capripoxvirus avec les sérums à analyser est à la fois sensible et spécifique, mais elle est coûteuse et difficile à mettre en œuvre. L'utilisation en ELISA indirect de cet antigène exprimé par un vecteur approprié, constitue une épreuve sérologique de référence acceptable. Bowden T.R et al. (Journal of Virological Methods, 161, 2009 : 19-29) ont conduit une étude pour identifier et développer d'autres antigènes candidats spécifiques des Capripoxvirus pour la mise en œuvre de ces méthodes ELISA indirect.

Hong et al, 2010. Virol Journal, 7(1), 245, décrivent également une méthode ELISA indirect (antigène primaire synthétique P32 et anticorps Ilaire de chèvre anti-humain IgG-HRP) pour détecter des anticorps dans des moutons immunisés expérimentalement.

La demande WO2009048769 décrit également l'utilisation d'un kit d'ELISA indirect (antigène H3L + anticorps II^{aire} anti-espèce) pour tester le titre d'anticorps de souris utilisées pour fabriquer des anticorps.

Mais il subsiste le besoin de disposer de nouvelles méthodes de dosage immunologique, qui soient faciles à mettre en œuvre et standardisables, avec une sensibilité et spécificité améliorées vis-à-vis des virus de la famille des *Poxviridae* et en particulier vis-à-vis des Capripoxvirus, et permettant de détecter et/ou quantifier la présence de complexe antigène/anticorps, même après plusieurs semaines voire plusieurs mois post-infection et/ou post-vaccination. On sait que les anticorps apparaissent environ 15 jours après la vaccination et leur quantité augmente jusqu'à 30 jours avant de décroître jusqu'à des niveaux difficilement détectables avec les méthodes actuelles.

C'est dans ce contexte que la Demanderesse a testé différents antigènes candidats spécifiques en particulier des Capripoxvirus et différentes techniques, et mis au point une méthode de dosage immunologique répondant à ce besoin. Elle a ainsi développé une méthode de dosage immunologique à double antigène, distincte des méthodes ELISA indirect de l'art antérieur, mettant en œuvre comme antigène cible une protéine synthétique ou recombinante rP32 spécifique des Capripoxvirus (homologue de protéine H3L chez les Orthopoxvirus), permettant à partir d'un échantillon biologique d'animal susceptible d'avoir été infecté et/ou vacciné, de détecter et/ou quantifier la présence d'anticorps contre ledit virus, et ce, même après 5 mois post-vaccination, ce que ne permet aucune autre technologie disponible sur le marché avec la même sensibilité et/ou spécificité (test de neutralisation du virus, dosages immunologiques directs ou indirects), alors même que l'on peut supposer l'animal protégé.

Par ailleurs, la méthode de dosage immunologique selon l'invention présente en outre de nombreux avantages par rapport aux techniques de séroneutralisation et test immunopéroxydase indirect utilisées jusqu'ici.

En effet, lesdites techniques de séroneutralisation et test immunopéroxydase indirect de l'art antérieur :
- nécessitent la manipulation du virus, qui ne peut se faire qu'en zone confinée, et implique donc d'avoir accès aux dites zones, ce qui restreint la possibilité de diffusion à large échelle de la technique analytique.
- permettent d'obtenir les résultats sous un délai de 24 à 48h
- font appel à des critères subjectifs (lecture d'un opérateur) pour l'interprétation du résultat par échantillon,
- sont difficilement standardisables, et
- ne sont pas ou peu adaptées à l'analyse d'un large nombre d'échantillons et ne sont pas automatisables

Avantageusement, la méthode de dosage immunologique selon l'invention :
- peut être mise en œuvre dans tout laboratoire de sérologie,
- permet l'obtention des résultats dans un délai inférieur à 3 heures,
- fait appel à des critères objectifs (calcul à partir de valeurs de densités optiques) pour l'interprétation individuelle des échantillons testés,
- est standardisée via l'introduction dans chaque essai d'un témoin ou contrôle positif,
- est adaptée à l'analyse d'un grand nombre d'échantillons et automatisable,
- est exclusive d'une famille (genre) de virus donnée (pas de réaction croisée entre une protéine P32 d'une famille de virus et un échantillon biologique infecté par un virus d'une autre famille).

### RESUME DE LA DESCRIPTION ET DE L'INVENTION

La présente invention concerne les objets tels que définis dans les revendications 1 à 10.

Un premier objet de la description est donc une méthode de dosage immunologique d'un anticorps spécifique d'un antigène d'un virus appartenant à la famille des *Poxviridae,* en particulier du genre Orthopoxvirus, du genre Parapoxvirus ou préférentiellement selon l'invention du genre Capripoxvirus, dans un échantillon biologique comprenant les étapes suivantes :
(i) Mise en contact d'au moins un antigène primaire comprenant au moins une protéine synthétique ou recombinante rP32 ou un fragment peptidique antigénique de celle-ci, avec un échantillon biologique suspecté de contenir ledit anticorps spécifique dudit antigène primaire, dans des conditions permettant la formation d'un complexe antigène primaire / anticorps' ;
(ii) Ajout d'au moins un conjugué contenant un antigène secondaire comprenant au moins une protéine synthétique ou recombinante rP32 ou un fragment peptidique antigénique de celle-ci et un marqueur dans des conditions permettant la formation d'un complexe antigène primaire / anticorps / conjugué' ; et
(iii) Détection dudit complexe antigène primaire / anticorps/ conjugué'.

De préférence, l'antigène primaire à l'étape (i) est fixé sur un support solide.

Selon un mode particulier, l'échantillon biologique sera un échantillon biologique d'un animal susceptible d'avoir été infecté et/ou vacciné depuis plus de 1 mois, voire 2 mois, de préférence depuis plus de 3 mois, de préférence encore depuis plus de 4 mois, voire plus de 5 mois. Selon un mode préféré, l'échantillon biologique sera un échantillon de sérum ou plasma d'un animal bovin, caprin, ovin ou toute autre espèce sensible susceptible d'avoir été vacciné depuis plus de 3 mois, voire plus de 5 mois.

Un autre objet de la description est un kit de détection et/ou quantification d'un anticorps spécifique d'un antigène d'un virus appartenant à la famille des *Poxviridae,* en particulier du genre Orthopoxvirus, du genre Parapoxvirus ou préférentiellement selon l'invention du genre Capripoxvirus, dans un échantillon biologique comprenant :
(i) Un support solide sensibilisé avec au moins un antigène primaire d'un virus appartenant à la famille des *Poxviridae,* en particulier du genre Orthopoxvirus, du genre Parapoxvirus ou préférentiellement selon l'invention du genre Capripoxvirus, comprenant au moins une protéine synthétique ou recombinante rP32 ou un fragment peptidique antigénique de celle-ci,
(ii) au moins un conjugué comprenant au moins un antigène secondaire d'un virus appartenant à la famille des *Poxviridae,* en particulier du genre Orthopoxvirus, du genre Parapoxvirus ou préférentiellement selon l'invention du genre Capripoxvirus, comprenant une protéine synthétique ou recombinante rP32 ou un fragment peptidique antigénique de celle-ci et un marqueur,
(iii) Et éventuellement des moyens de développer et détecter la présence du complexe antigène primaire / anticorps/ conjugué.

La description concerne également l'utilisation de la méthode de dosage immunologique ou du kit sus-décrits pour détecter et diagnostiquer *in vitro,* à partir d'un échantillon biologique, une infection provoquée par un virus appartenant à la famille des *Poxviridae,* en particulier du genre Orthopoxvirus, Parapoxvirus ou Capripoxvirus, préférentiellement Capripoxvirus selon l'invention tels que le virus de la dermatose nodulaire contagieuse (DNC), le virus de la variole caprine (VC) et le virus de la clavelée (VO), en particulier à un temps t d'au moins 2 à 3 mois post-infection.

En particulier, l'utilisation *in vitro* de la méthode de dosage immunologique ou du kit sus-décrits est avantageuse pour suivre dans le temps une vaccination contre un virus appartenant à la famille des *Poxviridae,* en particulier les Capripoxvirus selon l'invention tels que le virus de la dermatose nodulaire contagieuse, le virus de la variole caprine et le virus de la clavelée, en particulier à un temps t d'au moins 2 à 3 mois post-vaccination, ce que ne permettent pas les autres techniques disponibles sur le marché avec une telle sensibilité et spécificité.

### DESCRIPTION DETAILLEE DE LA DESCRIPTION ET DE L'INVENTION

### Méthode de dosage immunologique

La description concerne donc une méthode de dosage immunologique d'un anticorps spécifique d'un antigène d'un virus appartenant à la famille des *Poxviridae* , en particulier du genre Orthopoxvirus, du genre Parapoxvirus ou préférentiellement selon l'invention du genre Capripoxvirus, dans un échantillon biologique comprenant les étapes suivantes :
(i) Mise en contact d'au moins un antigène primaire comprenant au moins une protéine synthétique ou recombinante rP32 ou un fragment peptidique antigénique de celle-ci, avec un échantillon biologique suspecté de contenir ledit anticorps spécifique dudit antigène primaire, dans des conditions permettant la formation d'un complexe antigène primaire / anticorps' ;
(ii) Ajout d'au moins un conjugué contenant au moins un antigène secondaire comprenant une protéine synthétique ou recombinante rP32 ou un fragment peptidique antigénique de celle-ci et un marqueur, dans des conditions permettant la formation d'un complexe antigène primaire / anticorps / conjugué' ; et
(iii) Détection dudit complexe antigène primaire / anticorps/ conjugué'.

De préférence, ledit antigène primaire est fixé sur un support solide. Autrement dit, on sensibilise le support solide en le mettant en contact avec ledit antigène primaire.

Par 'virus appartenant à la famille des *Poxviridae',* on entend notamment les virus du genre Orthopoxvirus, tels que Cowpoxvirus et Camelpoxvirus, les virus du genre Parapoxvirus, et plus particulièrement selon l'invention les virus du genre Capripoxvirus tels que le virus de la dermatose nodulaire contagieuse (NDC), le virus de la variole caprine (VC) et le virus de la clavelée ou variole ovine (VO).

Par 'échantillon biologique', on entend notamment un échantillon de tissu ou de fluide corporel susceptibles de contenir des anticorps spécifiques dudit antigène cible (autrement nommé dans le reste de la description anticorps ou anticorps anti-antigène ou anticorps anti-CPV). Selon un mode particulier, ledit échantillon biologique est choisi parmi un échantillon de sang, sérum, plasma, lait, salive, expectoration, fluide cérébrospinal, ou exsudats de tissus, de fécès, de préférence un échantillon de sérum ou de plasma.

En particulier, ledit échantillon biologique est un échantillon de toute espèce animale sensible à un virus appartenant à la famille des *Poxviridae,* en particulier un mammifère, de préférence choisi dans le groupe constitué des bovins, des ovins, et des caprins, ou de toute autre espèce sensible, comme les girafes.

Selon un autre mode particulier, il s'agira d'un échantillon biologique humain.

On utilisera préférentiellement un échantillon biologique d'origine bovine, caprine, ovine choisi parmi le sérum ou le plasma.

Selon un mode particulier, l'échantillon biologique sera un échantillon biologique d'un animal susceptible d'avoir été infecté et/ou vacciné depuis plus de 1 mois voire plus de 2 mois, de préférence depuis plus de 3 mois, de préférence encore depuis plus de 4 mois, voire plus de 5 mois.

Selon un mode préféré, l'échantillon biologique sera un échantillon de sérum ou plasma d'un animal bovin, caprin, ovin, susceptible d'avoir été infecté et/ou vacciné depuis plus de 5 mois. L'échantillon biologique sera généralement obtenu par des méthodes classiques connues par l'homme du métier, par exemple au moyen d'une prise de sang.

Par dosage immunologique (à double antigène selon l'invention), on entend un dosage permettant de détecter la liaison de deux molécules d'antigène cible à l'anticorps spécifique desdits antigènes cibles, l'un d'entre eux faisant partie d'un conjugué comprenant ledit antigène cible et un marqueur.

Le dosage immunologique selon l'invention présente une spécificité et sensibilité améliorées par rapport aux autres techniques testées, comme cela est clairement illustré dans les exemples. Il présente en outre l'avantage de ne pas générer de réaction croisée entre différentes familles (genres) de virus. On peut en effet voir que la spécificité de l'essai immunologique de l'invention appliqué à la détection d'anticorps spécifiques à la dermatose nodulaire contagieuse (DNC) est de 100% et la sensibilité est de 100 % dans les conditions classiques (infection et/ou vaccination de moins de 2 mois) et de 75% dans les conditions extrêmes (vaccination de plus de 5 mois) alors qu'on ne dépasse pas les 36% avec les autres tests.

La sensibilité améliorée semble être due au fait que le dosage immunologique à double antigène de l'invention combine les deux avantages principaux des tests indirects et compétitifs ; en effet, il est capable de détecter tout type d'anticorps qui se lie à un antigène cible (et non seulement les anticorps se liant à un certain épitope, comme dans les dosages par compétition) et, d'autre part, comme dans les essais de compétition, de grandes quantités d'échantillon (sans dilution ou peu diluée) peuvent être utilisées dans l'essai, ce qui favorise la détection des points positifs faibles.

De même, la grande spécificité du dosage immunologique de l'invention semble être due au fait que l'antigène cible doit être reconnu deux fois par le même anticorps.

La méthode selon l'invention présente donc une exclusivité vis-à-vis du genre de virus recherché : une protéine P32 d'un virus d'une famille (genre) donnée (ex : Capripoxvirus) ne réagira pas avec un sérum infecté par un virus d'une autre famille (genre) donnée (ex : Parapoxvirus), contrairement aux autres méthodes disponibles.

Enfin le choix sélectif selon l'invention de la protéine P32 (synthétique ou recombinante) de Capripoxvirus, ou son homologue H3L chez les Orthopoxvirus selon la description, comme antigène CPV (Capripoxvirus) selon l'invention (ou OPV pour Orthopoxvirus selon la description), contribue à ces performances, comparativement aux autres antigènes CPV candidats testés.

L'antigène cible utilisé dans la présente description spécifique des virus de la famille des *Poxviridae,* en particulier des genres Orthopoxvirus (OPV) et Capripoxvirus (CPV), et préférentiellement selon l'invention du genre Capripoxvirus, autrement nommé antigène CPV, est choisi parmi une protéine synthétique ou recombinante rP32 selon l'invention (homologue de H3L chez les Orthopoxvirus selon la description) ou un fragment peptidique antigénique de celle-ci.

Dans la description, l'expression protéine P32' (recombinante ou synthétique) est utilisée pour désigner selon l'invention la protéine P32 de Capripoxvirus (telle que la protéine P32 de Lumpy Skin Disease Virus illustrée par la séquence SEQ ID N°1 décrite ci-après), mais aussi selon la description ses homologues H3L exprimées chez les Orthopoxvirus, dont les Cowpoxvirus et Camelpoxvirus, respectivement illustrées dans les séquences SEQ ID N°4 et SEQ ID N°5 décrites ci-après.

Ces protéines homologues sont généralement des protéines dont les gènes qui les codent ont une origine commune, dont les structures spatiales sont proches et dont les séquences en acides aminés présentent des similarités en lien avec leur fonction.

La littérature scientifique et en particulier les sites NCBI et Uniprot référencent les correspondances des protéines P32 des Capripoxvirus selon l'invention et des protéines H3L des Orthopoxvirus selon la description (appelées de ce fait protéines 'homologues' dans la présente description).

On parlera indifféremment dans le reste de la description d'antigène CPV (Capripoxvirus) ou antigène LSD (Lumpy Skin Disease) ou antigène P32 ou protéine P32 ou protéine synthétique ou recombinante rP32 ou un fragment peptidique antigénique de celle-ci, englobant également selon la description les expressions antigène OPV (Orthopoxvirus) et les protéines P32 homologues chez les Orthopoxvirus, autrement nommées protéine H3L.

L'homme du métier saura choisir la protéine P32 (synthétique ou recombinante, protéine totale ou fragment antigénique) à utiliser dans la méthode selon l'invention de dosage immunologique d'un anticorps spécifique d'un antigène d'un virus donné, en fonction de la famille (genre) du virus cible recherché dans les échantillons biologiques testés.

Ainsi, selon un mode particulier et préféré de l'invention, il utilisera dans la méthode selon l'invention de dosage immunologique d'un anticorps spécifique d'un antigène d'un virus du genre Capripoxvirus, la protéine P32 de Capripoxvirus, en particulier la protéine P32 de Lumpy Skin Disease Virus illustrée par la séquence SEQ ID N°1 décrite ci-après).

Selon un autre exemple de la description, il utilisera dans la méthode de dosage immunologique d'un anticorps spécifique d'un antigène d'un virus du genre Cowpoxvirus, une protéine H3L (homologue de P32 exprimée chez les Orthopoxvirus, dont les Cowpoxvirus et Camelpoxvirus), en particulier la protéine H3L illustrée par la séquence SEQ ID N°4 décrite ci-après.

Selon un autre exemple particulier de la description, il utilisera dans la méthode de dosage immunologique d'un anticorps spécifique d'un antigène d'un virus du genre Camelpoxvirus, une protéine H3L illustrée par la séquence SEQ ID N°5 décrite ci-après.

On utilisera ainsi au moins un antigène P32 selon l'invention (ou H3L selon la description) comme antigène primaire.

Selon un mode particulier, on utilisera une combinaison d'un antigène P32 selon l'invention (ou H3L selon la description) avec un autre antigène CPV selon l'invention (OPV selon la description) distinct (autre que rP32), afin notamment d'améliorer et/ou augmenter la sensibilité et/ou spécificité de la méthode sur le plus grand nombre d'échantillons biologiques.

Il peut également s'agir d'une protéine de fusion comprenant au moins protéine synthétique ou recombinante rP32 selon l'invention (ou rH3L selon la description) ou un fragment peptidique antigénique de celle-ci et une autre protéine synthétique ou recombinante d'un autre antigène candidat CPV selon l'invention (OPV selon la description) distinct ou un fragment de celui-ci. Les fragments antigéniques de la protéine P32 selon l'invention (ou H3L selon la description) peuvent être utilisés à la place de la protéine P32 (ou H3L), à condition que lesdits fragments soient capables de se lier à des anticorps dirigés contre les Capripoxvirus selon l'invention (ou Orthopoxvirus selon la description), ou des protéines de fusion comprenant au moins une desdites protéines ou desdits fragments antigéniques de celle-ci.

L'homme du métier, sur la base de ses connaissances et des méthodes à disposition, pourra ainsi, en exprimant des parties de la séquence ou en réalisant un épitope mapping par synthèse de peptides chevauchants, définir des fragments antigéniques utilisables selon l'invention. On peut citer par exemple la méthode décrite dans Reineke U, Sabat R. 'Antibody epitope mapping using SPOT peptide arrays'. Methods Mol Biol. 2009;524:145-67. doi: 10.1007/978-1-59745-450-6_11. PubMed PMID: 19377943.

Selon un mode particulier, les fragments antigéniques comprennent une séquence d'au moins 10 acides aminés de la séquence en acides aminés de la protéine P32 selon l'invention (H3L selon la description), notamment de 50 à 320 acides aminés, et en particulier de 200 à 320 acides aminés.

De même que lesdites protéines, les fragments antigéniques peuvent être d'origine synthétique ou recombinante.

La protéine rP32 selon l'invention (rH3L selon la description) ou fragments antigéniques de celle-ci peuvent avoir une ou plusieurs variations d'acides aminés conservatrices (de propriétés similaires) ou d'autres modifications mineures (ex : mutations), et conservent l'activité biologique de liaison à l'anticorps spécifique dudit antigène CPV selon l'invention (OPV selon la description) cible, aisément vérifiable par un test immunologique de détection du complexe antigène/anticorps. On parlera notamment de séquences dérivées ou mutées de ladite protéine rP32 selon l'invention (rH3L selon la description) ou fragments antigéniques de celles-ci.

Selon un mode particulier de la description, l'antigène primaire comprend au moins une séquence en acides aminés choisie dans le groupe constitué par :
a) la séquence SEQ ID N° 1, la séquence SEQ ID N°4, la séquence SEQ ID N°5 ou une séquence présentant au moins 90% d'identité, de préférence au moins 95% d'identité respectivement avec la SEQ ID N°1, la séquence SEQ ID N°4, ou la séquence SEQ ID N°5 ou
b) un fragment peptidique antigénique d'au moins 10 acides aminés de la SEQ ID N° 1, de la séquence SEQ ID N°4, de la séquence SEQ ID N°5 ou une séquence présentant au moins 90% d'identité, de préférence au moins 95% d'identité avec ledit fragment peptidique antigénique.

Selon un mode préféré de l'invention, en particulier pour détecter une infection ou suivre une vaccination à un virus du genre Capripoxvirus, l'antigène primaire selon l'invention comprend au moins une séquence en acides aminés choisie parmi la séquence SEQ ID N° 1 (protéine P32 de Capripoxvirus CPV) ou une séquence présentant au moins 90% d'identité, de préférence au moins 95% d'identité avec la SEQ ID N°1 ou un fragment peptidique antigénique d'au moins 10 acides aminés de la SEQ ID N° 1 ou une séquence présentant au moins 90% d'identité, de préférence au moins 95% d'identité avec ledit fragment peptidique antigénique. Selon un mode particulier, l'antigène primaire comprend au moins la séquence en acides aminés SEQ ID N°1 ou une séquence présentant au moins 90% d'identité, de préférence au moins 95% d'identité avec la SEQ ID N°1.

Selon un autre mode particulier de l'invention, l'antigène primaire comprend au moins un fragment peptidique antigénique comprenant une séquence d'au moins 10 acides aminés de la séquence SEQ ID N°1, en particulier de 50 à 320 acides aminés et notamment de 200 à 320 acides aminés de la séquence SEQ ID N°1, ou une séquence présentant au moins 90% d'identité, de préférence au moins 95% d'identité avec ledit fragment antigénique.

Selon un mode particulier et préféré de l'invention, l'antigène primaire comprend au moins la séquence en acides aminés SEQ ID N°1.

Selon un autre exemple de la description, en particulier pour détecter une infection ou suivre une vaccination à un virus du genre Cowpoxvirus, l'antigène primaire comprend au moins une séquence en acides aminés choisie parmi la séquence SEQ ID N° 4 (protéine P32 de Cowpoxvirus CWPV, autrement nommée protéine H3L) ou une séquence présentant au moins 90% d'identité, de préférence au moins 95% d'identité avec la SEQ ID N°4 ou un fragment peptidique antigénique d'au moins 10 acides aminés de la SEQ ID N° 4 ou une séquence présentant au moins 90% d'identité, de préférence au moins 95% d'identité avec ledit fragment peptidique antigénique.

Selon un exemple particulier de la description, l'antigène primaire comprend au moins la séquence en acides aminés SEQ ID N° 4 ou une séquence présentant au moins 90% d'identité, de préférence au moins 95% d'identité avec la SEQ ID N° 4.

Selon un autre exemple particulier de la description, l'antigène primaire comprend au moins un fragment peptidique antigénique comprenant une séquence d'au moins 10 acides aminés de la séquence SEQ ID N° 4, en particulier de 50 à 320 acides aminés et notamment de 200 à 320 acides aminés de la séquence SEQ ID N° 4, ou une séquence présentant au moins 90% d'identité, de préférence au moins 95% d'identité avec ledit fragment antigénique.

Selon un exemple particulier et préféré de la description, l'antigène primaire comprend au moins la séquence en acides aminés SEQ ID N°4.

Selon un autre exemple de la description, en particulier pour détecter une infection ou suivre une vaccination à un virus du genre Camelpoxvirus l'antigène primaire comprend au moins une séquence en acides aminés choisie parmi la séquence SEQ ID N° 5 (protéine P32 de Camelpoxvirus CMPV, autrement nommée protéine H3L) ou une séquence présentant au moins 90% d'identité, de préférence au moins 95% d'identité avec la SEQ ID N° 5 ou un fragment peptidique antigénique d'au moins 10 acides aminés de la SEQ ID N° 5 ou une séquence présentant au moins 90% d'identité, de préférence au moins 95% d'identité avec ledit fragment peptidique antigénique.

Selon un exemple particulier de la description, l'antigène primaire comprend au moins la séquence en acides aminés SEQ ID N° 5 ou une séquence présentant au moins 90% d'identité, de préférence au moins 95% d'identité avec la SEQ ID N° 5.

Selon un autre exemple particulier de la description, l'antigène primaire comprend au moins un fragment peptidique antigénique comprenant une séquence d'au moins 10 acides aminés de la séquence SEQ ID N° 5, en particulier de 50 à 320 acides aminés et notamment de 200 à 320 acides aminés de la séquence SEQ ID N° 5, ou une séquence présentant au moins 90% d'identité, de préférence au moins 95% d'identité avec ledit fragment antigénique.

Selon un mode particulier et préféré, l'antigène primaire comprend au moins la séquence en acides aminés SEQ ID N°5.

Le pourcentage d'identité entre deux séquences a une signification reconnue dans l'état de l'art ; il est déterminé sur la base d'un alignement global des séquences à comparer, c'est-à-dire sur un alignement des séquences prises dans leur intégralité sur toute la longueur en utilisant tout algorithme bien connu de l'homme du métier tel que l'algorithme de Needleman et Wunsch-1970 ; et l'homme du métier utilisera les méthodes connues de comparaison et d'alignement, par exemple les algorithmes de comparaison de séquences, pour mesurer l'identité entre deux séquences polypeptidiques. On peut citer à titre d'exemple les algorithmes BLAST disponibles sur les sites NCBI et Uniprot.

Par 'conjugué' selon l'invention, on entend un composé comprenant un antigène secondaire cible et un marqueur.

Selon un mode préféré, en particulier pour détecter une infection ou suivre une vaccination à un virus du genre Capripoxvirus, l'antigène primaire et l'antigène secondaire comprennent au moins une séquence en acides aminés choisie parmi la séquence SEQ ID N° 1 (protéine P32 de Capripoxvirus CPV) ou une séquence présentant au moins 90% d'identité, de préférence au moins 95% d'identité avec la SEQ ID N°1 ou un fragment peptidique antigénique d'au moins 10 acides aminés de la SEQ ID N° 1 ou une séquence présentant au moins 90% d'identité, de préférence au moins 95% d'identité avec ledit fragment peptidique antigénique.

Selon un autre mode préféré, l'antigène primaire et l'antigène secondaire comprennent au moins un fragment peptidique antigénique comprenant une séquence d'au moins 10 acides aminés de la séquence SEQ ID N°1, en particulier de 50 à 320 acides aminés et notamment 200 à 320 acides aminés de la séquence SEQ ID N°1, ou une séquence présentant au moins 90% d'identité, de préférence au moins 95% d'identité avec ledit fragment antigénique. Selon un mode préféré, l'antigène primaire et l'antigène secondaire comprennent la même séquence en acides aminés, l'antigène secondaire (conjugué) comprenant en outre un marqueur.

Selon un mode particulier et préféré, l'antigène primaire et l'antigène secondaire comprennent au moins la séquence en acides aminés SEQ ID N°1, l'antigène secondaire (conjugué) comprenant en outre un marqueur.

Selon un autre exemple de la description, en particulier pour détecter une infection ou suivre une vaccination à un virus du genre Cowpoxvirus, l'antigène primaire et l'antigène secondaire comprennent au moins une séquence en acides aminés choisie parmi la séquence SEQ ID N° 4 (protéine P32 de Cowpoxvirus CWPV, autrement nommée protéine H3L) ou une séquence présentant au moins 90% d'identité, de préférence au moins 95% d'identité avec la SEQ ID N°4 ou un fragment peptidique antigénique d'au moins 10 acides aminés de la SEQ ID N° 4 ou une séquence présentant au moins 90% d'identité, de préférence au moins 95% d'identité avec ledit fragment peptidique antigénique.

Selon un exemple particulier de la description, l'antigène primaire et l'antigène secondaire comprennent au moins un fragment peptidique antigénique comprenant une séquence d'au moins 10 acides aminés de la séquence SEQ ID N°4, en particulier de 50 à 320 acides aminés et notamment 200 à 320 acides aminés de la séquence SEQ ID N°4, ou une séquence présentant au moins 90% d'identité, de préférence au moins 95% d'identité avec ledit fragment antigénique.

Selon un exemple préféré de la description, l'antigène primaire et l'antigène secondaire comprennent la même séquence en acides aminés, l'antigène secondaire (conjugué) comprenant en outre un marqueur.

Selon un exemple particulier et préféré de la description, l'antigène primaire et l'antigène secondaire comprennent au moins la séquence en acides aminés SEQ ID N°4, l'antigène secondaire (conjugué) comprenant en outre un marqueur.

Selon un autre exemple particulier de la description, en particulier pour détecter une infection ou suivre une vaccination à un virus du genre Camelpoxvirus l'antigène primaire et l'antigène secondaire comprennent au moins une séquence en acides aminés choisie parmi la séquence SEQ ID N° 5 (protéine P32 de Camelpoxvirus CMPV, autrement nommée protéine H3L) ou une séquence présentant au moins 90% d'identité, de préférence au moins 95% d'identité avec la SEQ ID N°5 ou un fragment peptidique antigénique d'au moins 10 acides aminés de la SEQ ID N° 5 ou une séquence présentant au moins 90% d'identité, de préférence au moins 95% d'identité avec ledit fragment peptidique antigénique.

Selon un exemple particulier de la description, l'antigène primaire et l'antigène secondaire comprennent au moins un fragment peptidique antigénique comprenant une séquence d'au moins 10 acides aminés de la séquence SEQ ID N°5, en particulier de 50 à 320 acides aminés et notamment 200 à 320 acides aminés de la séquence SEQ ID N°5, ou une séquence présentant au moins 90% d'identité, de préférence au moins 95% d'identité avec ledit fragment antigénique.

Selon un exemple préféré de la description, l'antigène primaire et l'antigène secondaire comprennent la même séquence en acides aminés, l'antigène secondaire (conjugué) comprenant en outre un marqueur.

Selon un exemple particulier et préféré de la description, l'antigène primaire et l'antigène secondaire comprennent au moins la séquence en acides aminés SEQ ID N°5, l'antigène secondaire (conjugué) comprenant en outre un marqueur.

Le terme 'marqueur' se rapporte à un réactif indicateur qui permet de détecter le complexe antigène/anticorps/conjugué.

En particulier, ledit marqueur peut être choisi dans le groupe constitué d'une enzyme, un composé fluorescent ou un fluorophore, un composé (chimio)luminescent, un élément radioactif, une particule d'or colloïdale ou de latex.

A titre d'exemples, on peut citer :
- Pour les enzymes : la peroxydase, la glucosidase, la phosphatase alcaline, la glucose-6-phosphate déshydrogénase, la β-galactosidase, β-glucosidase, la β-glucuronidase,
- Pour les composés fluorescents ou fluorophores : la fluorescéine, la rhodamine,
- Pour les composés (chimio)luminescents : des dioxétanes, acridiniums, phenanthridiniums, le ruthénium, le luminol,
- Pour les éléments radioactifs : le soufre, l'iode,
- Pour les particules : or colloïdal, latex.

De préférence, il s'agira d'une enzyme, de préférence encore d'une enzyme choisie parmi la peroxydase et la phosphatase alcaline.

Le conjugué comprenant ledit antigène cible et ledit marqueur peut être obtenu par des méthodes classiques connues par l'homme du métier.

Le complexe antigène / anticorps / conjugué formé peut être détecté par toute technique appropriée, en fonction du marqueur choisi, par exemple au moyen de techniques basées sur la colorimétrie, la fluorométrie, la chimioluminescence, les méthodes radioactives, connues par l'homme du métier.

A titre d'exemple, lorsque le marqueur est une enzyme, la détection du complexe antigène/anticorps/conjugué peut être effectuée par mise en contact dudit complexe avec un substrat approprié, et éventuellement avec des agents appropriés d'amplification enzymatique et / ou d'activateurs.

A titre d'exemples, on peut citer comme substrat approprié et/ou agents appropriés d'amplification enzymatique et / ou d'activateurs:
- pour la phosphatase alcaline: substrats à base de phosphate de p-nitrophényle (p-NPP), 5-bromo-4-chloro-3-indolyl phosphate / nitrobleu de tétrazolium (BCIP / NBT) ; phosphate de 4-methylumbelliphenyl (4-MUP), 2- (5'-chloro-2'-phosphoryloxyphenyl) - 6-chloro-4- (3H) -quinazolinone (CPPCQ), 3, 6-diphosphate de fluorescéine (3 , 6-FDP) ;
- pour les peroxydases: substrats à base de 2,2-azinobis (3-éthylbenzothiazoline-6-sulfonique) (ABTS), l'acide o-phénylènediamine (OPD), l'acide 3,3 ', 5,5'-tétraméthylbenzidine (TMB), l'o-dianisidine, 5-aminosalicylique, l'acide 3-diméthylaminobenzoïque (DMAB), et l'acide 3-méthyl-2-benzothiazolinehydrazone (MBTH), le 3-amino-9-éthylcarbazole (AEC) et le 3,3'-diaminobenzidine (DAB), le tétrachlorure ; acide 4-hydroxy-3-méthoxyphénylacétique, les phénoxazines et benzothiazines réduite,
- pour glycosidases: substrats à base d'o-nitrophényl-β-D-galactoside (o-NPG), le p-nitrophényl-β-D-galactoside et de 4-methylumbelliphenyl-β-D-galactoside (MUG) pour la β-galactosidase ; β-D-galactopyranoside de résorufine, digalactoside fluorescéine (FDG), diglucuronide fluorescéine, le 4-méthylumbelliferyl bêta-D-galactopyranoside, carboxyumbelliferyl bêta-D-galactopyranoside, bêta-D-galactopyranosides fluorés coumarine, etc.

Dans un mode de réalisation particulier, ledit marqueur est une peroxydase telle que la peroxydase de raifort et le substrat chromogène approprié qui développe une couleur en présence de la peroxydase est le 5,5'-tétraméthylbenzidine (TMB).

Le dosage immunologique peut être choisi parmi le groupe constitué d'un dosage par technique immunochromatographique, un dosage immunoenzymatique (ELISA), un essai radioimmunologique (RIA), un dosage d' hémagglutination (HA), un test western blot, un test immunologique par polarisation de fluorescence (FPIA), et un test d'immunofluorescence indirecte.

Selon un mode particulier, le dosage immunologique est un dosage par immunochromatographie ou un dosage enzymo-immunologique en phase solide (ELISA), de préférence un dosage enzymo-immunologique en phase solide (ELISA).

La détection à double antigène selon l'invention, d'anticorps viraux par enzymo-immunologie en phase solide (ELISA) consiste à sensibiliser un puits par un antigène primaire CPV, déposer l'échantillon biologique à tester dans des conditions permettant la formation du complexe antigène/anticorps, puis appliquer le conjugué comprenant l'antigène secondaire et un marqueur (enzyme) pour la formation d'un complexe antigène/anticorps/conjugué, que l'on révèle en présence d'un substrat approprié.

La détection rapide à double antigène selon l'invention, d'anticorps viraux par immunochromatographie sur membrane consiste à déposer l'échantillon biologique à tester à l'une des extrémités d'une membrane de nitrocellulose fixée sur un support plastique ou carton. Si l'anticorps recherché est présent, il se lie avec un antigène primaire CPV et un conjugué comprenant un antigène secondaire CPV marqué le plus souvent à l'or colloïdal. Sous l'effet d'un tampon de migration, les complexes anticorps - antigène secondaire conjugué migrent par capillarité et sont arrêtés par des antigènes primaires CPV fixés sur la membrane. Un résultat positif se traduit par l'apparition d'une ligne colorée. L'excès d'antigène secondaire conjugué continue à migrer et est immobilisé par un anticorps anti CPV), l'accumulation des complexes colorés entraîne l'apparition d'une ligne colorée, cette seconde ligne ou ligne de contrôle valide le bon fonctionnement de la réaction. En cas de réaction négative, seule la ligne contrôle est colorée. L'apparition des bandes est rapide : en 15 à 20 mn. Le principe de la détection rapide d'anticorps par immunochromatographie sur bandelette est identique.

Le dosage immunologique peut être utilisé pour détecter et quantifier des anticorps spécifiques d'un antigène cible dans un échantillon biologique, puisque dans le cas de marqueurs comme les enzymes, la quantité d'anticorps présente dans l'échantillon de test est proportionnelle au signal généré.

L'antigène CPV cible est directement ou indirectement immobilisé sur un support solide.
Par 'support solide', on entend notamment un puits d'une plaque de microtitration, une bille magnétique, une bille non magnétique, une colonne, une matrice, une membrane, une bandelette, une natte fibreuse composée de fibres synthétiques ou naturelles (par exemple des matériaux en verre ou à base de cellulose ou des polymères thermoplastiques tels que le polyéthylène, le polypropylène ou polyester), une structure frittée composée de matériaux particulaires (par exemple, de verre ou de divers polymères thermoplastiques), ou d'un film de membrane synthétique composée par exemple de nitrocellulose ou nylon.

L'immobilisation de l'antigène cible sur ledit substrat ou support peut être réalisée de manière passive (via des liaisons ioniques, hydrophobes, etc...) ou covalentes (exemple via des réactions chimiques entre l'antigène et le support, directement ou via des agents de couplage).

Selon un mode particulier, on utilisera comme support solide pour un dosage enzymo-immunologique (ELISA) une plaque multi-puits de microtitration.

Selon un autre mode particulier, on utilisera comme support solide pour un dosage par immunochromatographie une membrane ou une bandelette, en particulier de nitrocellulose. Les tests immuno-chromatographiques sur bandelette (ICB), sont plus communément connus sous leur appellation anglaise de « *Lateral Flow Test».*

Dans un mode de réalisation particulier, l'antigène cible est directement ou indirectement immobilisée sur un support, tel qu'un puits d'une microplaque. L'échantillon biologique suspecté de contenir des anticorps spécifiques dudit antigène cible CPV est incubé avec ledit antigène cible pendant une période de temps et dans des conditions appropriées pour que des complexes antigène/anticorps se forment. Après avoir lavé le puits pour éliminer les réactifs non liés à l'antigène, un conjugué comprenant ledit antigène cible lié à un marqueur est ajouté et on laisse incuber pendant une période de temps et dans des conditions appropriées à la formation du complexe antigène/anticorps/conjugué. La détection desdits complexes antigène/anticorps/conjugué en présence d'un substrat approprié est indicatrice de la présence et/ou de la quantité d'anticorps spécifiques contre ledit antigène cible dans l'échantillon biologique suspecté d'en contenir.

Selon un mode particulier et préféré de la description, la méthode de dosage immunologique à double antigène d'un anticorps spécifique d'un antigène d'un virus appartenant à la famille des *Poxviridae,* en particulier du genre Orthopoxvirus ou Capripoxvirus, préférentiellement Capripoxvirus selon l'invention, dans un échantillon biologique est un dosage enzymo-immunologique à double antigène ELISA réalisé sur des plaques de microtitration selon les étapes suivantes :
- Les puits de la microplaque de microtitration sont sensibilisés avec l'antigène purifié de CPV (Capripoxvirus) selon l'invention ou OPV (Orthopoxvirus) selon la description;
- Les échantillons biologiques à tester et les témoins sont ajoutés aux micro-puits ; les anticorps anti-CPV selon l'invention (OPV selon la description), s'ils sont présents, forment un complexe anticorps-antigène ;
- Les plaques sont ensuite lavées et le conjugué, un antigène purifié de CPV marqué avec de la peroxydase (HRP), est ajouté aux micro-puits. Il fixe le Fab libre des anticorps anti-CPV selon l'invention (OPV selon la description) sériques liés ;
- Après lavage afin d'éliminer l'excès de conjugué, on ajoute la solution de substrat (par exemple TMB : Tetramethylbenzidine) puis la réaction est arrêtée par ajout d'une solution acide.

La microplaque est ensuite lue à 450 nm sur un spectrophotomètre.

La coloration résultante dépend de la quantité d'anticorps spécifiques présents dans l'échantillon à tester: en présence d'anticorps, une solution bleue apparaît qui devient jaune après addition de la solution d'arrêt ; alors qu'en l'absence d'anticorps, aucune coloration n'apparaît.

### Kit

La description porte également sur un kit de détection et/ou quantification d'un anticorps spécifique d'un antigène d'un virus appartenant à la famille des *Poxviridae,* en particulier du genre Orthopoxvirus, du genre Parapoxvirus ou préférentiellement selon l'invention du genre Capripoxvirus, dans un échantillon biologique comprenant :
(i) Un support solide sensibilisé avec au moins un antigène primaire d'un virus appartenant à la famille des *Poxviridae,* en particulier du genre Orthopoxvirus, du genre Parapoxvirus ou préférentiellement selon l'invention du genre Capripoxvirus, comprenant au moins une protéine synthétique ou recombinante rP32 selon l'invention (rH3L selon la description) ou un fragment peptidique antigénique de celle-ci,
(ii) au moins un conjugué comprenant un antigène secondaire d'un virus appartenant à la famille des *Poxviridae,* en particulier du genre Orthopoxvirus, du genre Parapoxvirus ou préférentiellement selon l'invention du genre Capripoxvirus, comprenant au moins une protéine synthétique ou recombinante rP32 selon l'invention (rH3L selon la description) ou un fragment peptidique antigénique de celle-ci et un marqueur,
(iii) Et éventuellement des moyens de développer et détecter la présence du complexe antigène primaire / anticorps/ conjugué.

Avantageusement, le kit selon l'invention comprendra en outre au moins :
(iv) un contrôle choisi parmi un contrôle positif constitué d'un échantillon contenant des anticorps anti-CPV ;
(v) un contrôle négatif constitué d'un échantillon biologique ne contenant pas d'anticorps anti-CPV, et de préférence leur mélange.

Selon un exemple de la description, l'antigène primaire et le conjugué comprennent au moins une séquence en acides aminés choisie dans le groupe constitué par :
a) la séquence SEQ ID N° 1, la séquence SEQ ID N°4, la séquence SEQ ID N°5 ou une séquence présentant au moins 90% d'identité, de préférence au moins 95% d'identité respectivement avec la séquence SEQ ID N°1, la séquence SEQ ID N°4, ou la séquence SEQ ID N°5 ou
b) un fragment peptidique antigénique d'au moins 10 acides aminés de la séquence SEQ ID N° 1, la séquence SEQ ID N°4, la séquence SEQ ID N°5 ou une séquence présentant au moins 90% d'identité, de préférence au moins 95% d'identité avec ledit fragment peptidique antigénique, et le marqueur est une enzyme, de préférence la peroxydase.

Selon un mode particulier et préféré de l'invention, en particulier pour un kit de détection et/ou quantification d'un anticorps spécifique d'un antigène d'un virus du genre Capripoxvirus, l'antigène primaire et le conjugué comprennent au moins une séquence en acides aminés choisie parmi la séquence SEQ ID N° 1 ou une séquence présentant au moins présentant au moins 90% d'identité, de préférence au moins 95% d'identité avec la SEQ ID N°1 ou un fragment peptidique antigénique d'au moins 10 acides aminés de la SEQ ID N° 1 ou une séquence présentant au moins 90% d'identité, de préférence au moins 95% d'identité avec ledit fragment peptidique antigénique, et le marqueur est une enzyme, de préférence la peroxydase.

Selon un mode particulier et préféré, l'antigène primaire et le conjugué comprennent au moins une séquence en acides aminés choisie parmi la séquence SEQ ID N° 1 et le marqueur est la peroxydase.

Selon un autre exemple de la description, en particulier pour un kit de détection et/ou quantification d'un anticorps spécifique d'un antigène d'un virus du genre Cowpoxvirus, l'antigène primaire et le conjugué comprennent au moins une séquence en acides aminés choisie parmi la séquence SEQ ID N° 4 (protéine P32 de Cowpoxvirus CWPV, autrement nommée protéine H3L) ou une séquence présentant au moins présentant au moins 90% d'identité, de préférence au moins 95% d'identité avec la SEQ ID N°4 ou un fragment peptidique antigénique d'au moins 10 acides aminés de la SEQ ID N° 4 ou une séquence présentant au moins 90% d'identité, de préférence au moins 95% d'identité avec ledit fragment peptidique antigénique, et le marqueur est une enzyme, de préférence la peroxydase.

Selon un exemple particulier et préféré de la description, l'antigène primaire et le conjugué comprennent au moins une séquence en acides aminés choisie parmi la séquence SEQ ID N° 4 et le marqueur est la peroxydase.

Selon un exemple particulier de la description, en particulier pour un kit de détection et/ou quantification d'un anticorps spécifique d'un antigène d'un virus du genre Camelpoxvirus, l'antigène primaire et le conjugué comprennent au moins une séquence en acides aminés choisie parmi la séquence SEQ ID N° 5 (protéine P32 de Camelpoxvirus CMPV, autrement nommée protéine H3L) ou une séquence présentant au moins présentant au moins 90% d'identité, de préférence au moins 95% d'identité avec la SEQ ID N°5 ou un fragment peptidique antigénique d'au moins 10 acides aminés de la SEQ ID N° 5 ou une séquence présentant au moins 90% d'identité, de préférence au moins 95% d'identité avec ledit fragment peptidique antigénique, et le marqueur est une enzyme, de préférence la peroxydase.

Selon un exemple particulier et préféré de la description, l'antigène primaire et le conjugué comprennent au moins une séquence en acides aminés choisie parmi la séquence SEQ ID N° 5 et le marqueur est la peroxydase.

### Utilisations

La description porte également sur l'utilisation de la méthode de dosage sus-décrite ou du kit la mettant en œuvre pour détecter et diagnostiquer *in vitro* , à partir d'un échantillon biologique, une infection causée par un virus appartenant à la famille des *Poxviridae,* en particulier du genre Orthopoxvirus, Parapoxvirus ou Capripoxvirus, préférentiellement Capripoxvirus selon l'invention tels que le virus de la dermatose nodulaire contagieuse, le virus de la variole caprine et le virus de la clavelée, en particulier à un temps t d'au moins 2 à 3 mois post-infection. Les animaux pouvant être infectés indépendamment du développement de symptômes cliniques.

La description porte donc également sur une méthode de détection et/ou diagnostic *in vitro* d'une infection causée par un virus appartenant à la famille des *Poxviridae,* en particulier du genre Orthopoxvirus, Parapoxvirus ou Capripoxvirus, préférentiellement Capripoxvirus selon l'invention tels que le virus de la dermatose nodulaire contagieuse, le virus de la variole caprine et le virus de la clavelée, à partir d'un échantillon biologique d'un animal suspecté d'avoir été infecté, comprenant au moins une étape de dosage immunologique d'un anticorps spécifique d'un antigène P32 selon l'invention (H3L selon la description) dudit virus, telle que décrite précédemment selon l'invention, et comparaison avec un échantillon témoin (non infecté). Comme décrit précédemment, l'homme du métier choisira la protéine P32 (synthétique ou recombinante, protéine totale ou fragment antigénique) à utiliser dans la méthode selon l'invention de détection et/ou diagnostic *in vitro* d'une infection causée par un virus donné, en fonction de la famille (genre) du virus cible recherché dans les échantillons biologiques testés. Ainsi, selon un mode particulier et préféré de l'invention, il utilisera dans la méthode selon l'invention de détection et/ou diagnostic *in vitro* d'une infection causée par un virus du genre Capripoxvirus la protéine P32 de Capripoxvirus, en particulier la protéine P32 de Lumpy Skin Disease Virus illustrée par la séquence SEQ ID N°1.

Selon un autre exemple particulier de la description, il utilisera dans la méthode de détection et/ou diagnostic *in vitro* d'une infection causée par un virus du genre Cowpoxvirus la protéine H3L de Cowpoxvirus, en particulier la protéine illustrée par la séquence SEQ ID N°4.

Selon un autre exemple particulier de la description, il utilisera dans la méthode *in vitro* de détection et/ou diagnostic *in vitro* d'une infection causée par un virus du genre Camelpoxvirus la protéine H3L de Camelpoxvirus, en particulier la protéine illustrée par la séquence SEQ ID N°5.

Avantageusement, on utilise la méthode de dosage selon la description ou le kit la mettant en œuvre pour suivre dans le temps une vaccination contre un virus appartenant à la famille des *Poxviridae,* en particulier les Capripoxvirus selon l'invention tels que le virus de la dermatose nodulaire contagieuse, le virus de la variole caprine et le virus de la clavelée, en particulier à un temps t d'au moins 2 à 3 mois post-vaccination.

La description porte donc également sur une méthode *in vitro* de suivi de la vaccination d'un animal contre un virus appartenant à la famille des *Poxviridae,* en particulier du genre Orthopoxvirus, Parapoxvirus ou Capripoxvirus, préférentiellement Capripoxvirus selon l'invention tels que le virus de la dermatose nodulaire contagieuse, le virus de la variole caprine et le virus de la clavelée, à partir d'un échantillon biologique d'un animal suspecté d'avoir été vacciné, comprenant au moins une étape de dosage immunologique d'un anticorps spécifique d'un antigène P32 selon l'invention (H3L selon la description) dudit virus, telle que décrite précédemment selon l'invention, et comparaison avec un échantillon témoin (non vacciné ni infecté).

Comme décrit précédemment, l'homme du métier choisira la protéine P32 (synthétique ou recombinante, protéine totale ou fragment antigénique) à utiliser dans la méthode de suivi de la vaccination d'un animal contre un virus donné, en fonction de la famille (genre) du virus cible recherché dans les échantillons biologiques testés.

Ainsi, selon un mode particulier et préféré de l'invention, il utilisera dans la méthode de suivi de la vaccination d'un animal contre un virus du genre Capripoxvirus la protéine P32 de Capripoxvirus, en particulier la protéine P32 de Lumpy Skin Disease Virus illustrée par la séquence SEQ ID N°1.

Selon un autre exemple de la description, il utilisera dans la méthode de suivi de la vaccination d'un animal contre un virus du genre Cowpoxvirus la protéine H3L de Cowpoxvirus, en particulier la protéine illustrée par la séquence SEQ ID N°4.

Selon un autre exemple particulier de la description, il utilisera dans la méthode de suivi de la vaccination d'un animal contre un virus du genre Camelpoxvirus la protéine H3L de Camelpoxvirus, en particulier la protéine illustrée par la séquence SEQ ID N°5.

Avantageusement, la méthode de dosage immunologique à double antigène selon l'invention :
- peut être mise en œuvre dans tout laboratoire de sérologie,
- permet l'obtention des résultats dans un délai inférieur à 3 heures,
- fait appel à des critères objectifs (calcul à partir de valeurs de densités optiques) pour l'interprétation individuelle des échantillons testés,
- est standardisé via l'introduction dans chaque essai d'un témoin ou contrôle positif,
- est adapté à l'analyse d'un grand nombre d'échantillons et sont automatisables.

L'invention va désormais être illustrée par les exemples non limitatifs suivants.

### EXEMPLES

### EXEMPLE 1 : Evaluation de différents protéines et techniques pour la détection d'une infection et/ou une vaccination contre le virus LSDV _(Lumpy Skin Disease Virus)

### 1.1. Préparation de l'Antigène primaire I^{aire} (protéine P32) :

La protéine recombinante P32 (rP32) du LSDV (pour Lumpy Skin Disease Virus) est utilisée en tant qu'antigène CPV (Capripox Virus). Cette protéine a été produite par culture de bactéries (BL21 DH5) transformées avec le plasmide pET 20b+ (Ref N°69739-3, NOVAGEN) contenant la séquence du gène codant pour la P32 du CPV (SEQ ID N° 1) telle que définie précédemment. Ce plasmide contient aussi un gène de résistance à l'ampicilline pour la sélection des bactéries transformées et permet l'ajout d'un tag 6-His (His-His-His-His-His-His) sur la P32 du CPV pour permettre une purification d'affinité.

Après culture, le culot bactérien obtenu après centrifugation à 5000 g pendant 15 min est repris dans du tampon PBS (phosphate buffer saline) pH = 7. Les bactéries contenant l'antigène CPV sont ensuite lysées par sonication et par des cycles de congélation/décongélation. Après une centrifugation de 15000 g pendant 30 min, le culot contenant l'antigène CPV est repris avec du tampon PBS + 8 M urée pH 8,5 , filtré à 0,45 µm, puis purifié par chromatographie d'affinité. Les fractions contenant l'antigène CPV sont identifiées par électrophorèse en gel de polyacrylamide contenant du dodécysulfate de sodium. L'antigène est ensuite renaturé par des étapes de dialyses successives contre des tampons contenant des concentrations dégressives d'urée (PBS 4M urée, puis 2M urée, puis 1 M urée, puis PBS seul). Une étape complémentaire de purification par chromatographie d'exclusion stérique est alors réalisée afin d'obtenir la meilleure pureté possible. La fraction la plus pure de rP32 du CPV ainsi obtenue est utilisée à la fois comme agent de sensibilisation des plaques de polystyrène et comme conjugué pour le test ELISA, à des concentrations comprises entre 0,1 et 10 Pg/mL.

### 1.2. Préparation des autres antigènes primaires I^{aires} (protéine A33 et B5) :

Les protéines recombinantes rA33 et rB5 du CPV ont été préparées de la même manière que la rP32 en utilisant le plasmide pET 20b+ (Ref N°69739-3, NOVAGEN) contenant soit le gène de la protéine A33 (SEQ ID N°2) du LSDV ou la de protéine B5 (SEQ ID N°3) LSDV.

### 1.3. Préparation des conjugués

Pour l'utilisation de la protéine en tant que conjugué dans le dosage immunologique à double antigène selon l'invention, la rP32 du LSDV a été couplée avec la peroxydase (HRP) (Horse Radish peroxidase, ROCHE) en suivant la méthode décrite par Nakane & Kawaoi (1974, J. Histochem. Cytochem. 22: 1084-1091).

Pour les tests indirects (ELISA), le conjugué employé est un anticorps polyclonal (Pab) anti-IgG de bovin (ref A10-102P, Bethyl Laboratories, USA), ou anti-IgG de chèvre (ref A50-100P, Bethyl Laboratories, USA), ou anti-IgG de mouton (ref A130-101P, Bethyl Laboratories, USA).

### 1.4. Préparation des échantillons :

Afin de comparer les performances (sensibilité, spécificité) de différents tests (indirect ELISA et double antigène) pour la détection d'anticorps anti-CPV, un panel composé d'échantillons biologiques (susceptibles de contenir des anticorps anti-CPV), ainsi que des échantillons positifs et négatifs a été utilisé.

### - Echantillons positifs :

Les échantillons positifs qui ont été utilisés sont :
- Echantillon N°1 : sérum d'un bovin infecté expérimentalement par le LSDV, récolté 37 jours après infection ;
- Echantillon N°2 : sérum d'un bovin expérimentalement vacciné (récolté 50 jours après vaccination) puis infecté/éprouvé (collecté 26 jours après infection) ;
- Echantillon N° 3 : sérum d'un bovin non vacciné, infecté expérimentalement et récolté 26 jours après infection ;
- Echantillon N°15 : Sérum d'un bovin vacciné avec un vaccin atténué commercial (souche Neethling), puis infecté, récolté 6 semaines après infection.

Les échantillons susceptibles d'être positifs, à évaluer sont :
- Echantillons (N°4 à N° 14) de sérum issus de 11 bovins d'un cheptel laitier immunisé contre le virus LSDV avec un vaccin atténué commercial (souche Neethling), récoltés cinq mois après vaccination.

### - Echantillons négatifs :

32 échantillons négatifs issus de zone indemne (absence de Capripoxvirus) et où la vaccination n'est pas pratiquée (France) sont testés et servent d'échantillons témoins négatifs.

Pour chaque protéine testée, la valeur seuil de positivité est obtenue en triplant la moyenne de densité optique des échantillons témoins négatifs (valeur seuil = moyenne de densité optique des négatifs x3).

### 1.5. Protocoles de tests :

### 1.5.1. Dosage monocouche en présence d'immunoperoxidase (IPMA pour ImmunoPeroxidase Monolayer Assay)

Des cellules épithéliales de rein de bovin Madin-Darby (MDBK) sont cultivées dans des plaques de microtitration à 96 puits. Après lavage une fois avec une solution de sel équilibrée de Hanks, les cellules ont été inoculées avec le LSDV (Lumpy Skin Disease Virus) à une multiplicité d'infection de 1. Les plaques ont ensuite été incubées à 37 ° C dans une atmosphère de CO2 à 5% pendant 48 heures, fixées dans de l'acétone-éthanol froid pendant 20 minutes, recouvertes et stockées à -20 ° C jusqu'à leur utilisation (habituellement dans les 2 mois suivant la préparation). Avant d'ajouter les échantillons, les plaques IPMA ont été lavées une fois avec une solution saline tamponnée au phosphate (PBS pH 7,2). Les dilutions sérielles d'un facteur 2 de chaque échantillon sont ensuite ajoutées (2 puits / dilution), puis sont incubées pendant 45 minutes à 37 ° C. Après lavage 3 fois avec du PBS, 50 ul (0,5 µg / ml) d'anticorps polyclonal anti-espèce référencé ci-dessus conjugué à la peroxydase a été ajouté à tous les puits et les plaques ont été incubées à 37 ° C pendant 45 minutes. Après 3 lavages, 50 ml de substrat TMB précipitant (SIGMA-ALDRICH) sont ajoutés, puis la réaction se développe pendant 20 minutes avant que les plaques soient lavées une fois avec du PBS puis examinées au microscope optique.

### 1.5.2 VNT (Test de neutralisation du virus)

1) Les sérums sont inactivés pendant 30 minutes au bain-marie à 56°C ;
2) Des dilutions de 2 en 2 des sérums à tester sont faites dans du milieu de culture de cellules, en commençant par le sérum non dilué (cela donne une étape de dilution de séroneutralisation de 1/2 une fois mélangé à un volume égal de suspension virale). Les dilutions sont préparées dans une plaque à fond plat de microtitrage de 96 puits en utilisant, de façon optimale, 3 puits par dilution et 25 µl par puits. Des sérums témoins positifs et négatifs sont également inclus dans l'épreuve.
3) 25 µl du stock du virus LSD sont ajoutés dans chaque puits, à une dilution effectuée dans le milieu de culture et calculée pour fournir 100 DICT₅₀ (dose de virus infectant 50% de la culture cellulaire) par puits. Le virus devrait être ajouté à 2/3 des puits contenant le sérum, pour chaque dilution. Le troisième puits sert de témoin, contenant uniquement le sérum, et devrait recevoir 25 µl de milieu de culture au lieu du virus ;
4) Le virus restant est titré par 4 étapes de dilution en 10 fois en utilisant 25 µl par puits et au moins 4 puits par dilution ; 25 µl du milieu de culture sont ajoutés à chacun des puits pour compenser l'absence du sérum d'essai ;
5) Les plaques sont agitées brièvement et incubées 1 h à 37°C dans une atmosphère à 5 % de CO₂ ;
6) 100 µl, par exemple, de suspension de cellules MDBK (cellules épitheliales de rein de bovin Madin-Darby) ou OA3.TS (Ovine testis cell line) à 2 × 10⁵ cellules/ml sont ajoutés à chaque puits ;
7) Les plaques sont incubées pendant entre 3 et 14 jours à 37°C dans une atmosphère à 5 % de CO₂ ;
8) Les plaques sont lues au microscope pour l'effet cytopathique (ECP). L'épreuve est validée en vérifiant le titre du virus (qui devrait avoir une valeur de 100 DICT₅₀ avec des valeurs tolérées entre 50 et 200 DICT₅₀) et les sérums témoins. Le sérum positif standard devrait avoir une valeur divergeant au maximum de 0,3 log₁₀ de la valeur moyenne prédéterminée. Des lectures de chaque dilution de sérum testé devraient être faites en référence au puits ne contenant que du sérum témoin pour distinguer l'ECP viral d'un effet cytotoxique induit par le sérum ou par une contamination ;
9) Les résultats pour le sérum testé sont déterminés par la méthode de Spearman-Kärber comme la dilution de sérum qui a neutralisé le virus dans 50 % des puits ;
10) Un sérum négatif ne devrait donner aucune séroneutralisation à la plus faible dilution examinée (c'est-à-dire le sérum non dilué, équivalent à une dilution de 1/2 à l'étape de neutralisation).

### 1.5.3 ELISA Indirect (protéines P32, A33 et B5)

Des plaques en polystyrène 96 puits sont incubées avec la protéine rP32 ou la protéine rA33 ou la protéine B5 du LSDV pendant 18 heures à +4°C en tampon carbonate-BSA (bovine serum albumin), pH 9,6. Les plaques sont ensuite lavées avec du tampon PBS contenant 0,05% Tween^{®} 20, pH 7.4 puis saturées pendant 2h à température ambiante avec du tampon PBS avec 2% BSA contenant 0,05% Tween^{®} 20, pH 7.4. Les plaques qui ne seront pas immédiatement utilisées sont mises en sachet individuel et séchées.

Ensuite les sérums à tester sont ajoutés sur les plaques à une dilution au 1/100 dans du tampon PBS avec 2% BSA contenant 0,05% Tween^{®} 20, pH 7.4 et sont incubées pendant 60 minutes à température ambiante.

Après trois lavages avec du tampon PBS contenant 0,05% Tween^{®} 20, pH 7.4, les plaques sont incubées avec un anticorps polyclonal anti-espéce couplé à la péroxydase à une dilution prédéterminée pendant 30 minutes à température ambiante.

A la fin du temps d'incubation, les plaques sont à nouveau lavées trois fois avec du tampon PBS contenant 0,05% Tween^{®} 20, pH 7.4 et la réaction est développée pendant 15 minutes à température ambiante après l'ajout du substrat (TMB : Tetramethylbenzidine). Après avoir arrêté la réaction par l'ajout d'acide sulfurique à 0,5M, l'absorbance est lue à 450 nm sur un lecteur de plaques.

### 1.5.4 Double Antigène

Des plaques en polystyrène 96 puits sont incubées avec la protéine rP32 du LSDV pendant 18 heures à température ambiante en tampon carbonate-BSA (bovine serum albumin), pH 9,6. Les plaques sont ensuite lavées avec du tampon PBS contenant 0,05% Tween^{®} 20, pH 7.4 puis saturées pendant 2h à température ambiante avec du tampon PBS avec 2% BSA contenant 0,05% Tween^{®} 20, pH 7.4. Les plaques qui ne seront pas immédiatement utilisées sont mises en sachet individuel et séchées.

Ensuite les sérums à tester sont ajoutés sur les plaques à une dilution au ½ dans du tampon PBS avec 2% BSA contenant 0,05% Tween^{®} 20, pH 7.4 et sont incubées pendant 90 minutes à température ambiante.

Après trois lavages avec du tampon PBS contenant 0,05% Tween^{®} 20, pH 7.4, les plaques sont incubées avec le conjugué rP32-HRP à une dilution prédéterminée pendant 30 minutes à température ambiante.

A la fin du temps d'incubation, les plaques sont à nouveau lavées cinq fois avec du tampon PBS contenant 0,05% Tween^{®} 20, pH 7.4 et la réaction est développée pendant 15 minutes à température ambiante après l'ajout du substrat (TMB : Tetramethylbenzidine). Après avoir arrêté la réaction par l'ajout d'acide sulfurique à 0,5M, l'absorbance est lue à 450 nm sur un lecteur de plaques.

### 1.6 Résultats :

### 1.6.1 Sensibilité :

### 1.6.1.1. IPMA (Comparatif) :

**Tableau 1**

| **Echantillons** | **Description des échantillons** | **Statut** |
|---|---|---|
| **Echantillon N°1** | Infecté expérimentalement (37 jours après infection) | ***Positif*** |
| **Echantillon N°2** | Vacciné (50 jours après vaccination) et infecté expérimentalement (29 jours après infection) | ***Positif*** |
| **Echantillon N°3** | Infecté expérimentalement (29 jours après infection) | ***Positif*** |
| **Echantillon N°4** | Vacciné (5 mois après vaccination) | Négatif |
| **Echantillon N°5** | | Négatif |
| **Echantillon N°6** | | Négatif |
| **Echantillon N°7** | | Négatif |
| **Echantillon N°8** | | Négatif |
| **Echantillon N°9** | | Négatif |
| **Echantillon N°10** | | Négatif |
| **Echantillon N°11** | | Négatif |
| **Echantillon N°12** | | Négatif |
| **Echantillon N°13** | | Négatif |
| **Echantillon N°14** | | Négatif |
| **Echantillon N°15** | Vacciné et naturellement infecté (6 semaines après infection) | Négatif |

Ces résultats montrent que la technologie IPMA ne permet pas de détecter d'anticorps de CPV dans des échantillons biologiques vaccinés depuis 5 mois.

### 1.6.1.2. VNT (Test de Neutralisation du virus) (comparatif):

**Tableau 2**

| **Echantillons** | **Description des échantillons** | **Statut** |
|---|---|---|
| **Echantillon N°1** | Infecté expérimentalement (37 jours après infection) | ***Positif*** |
| **Echantillon N°2** | Vacciné (50 jours après vaccination) et infecté expérimentalement (29 jours après infection) | ***Positif*** |
| **Echantillon N°3** | Infecté expérimentalement (29 jours après infection) | ***Positif*** |
| **Echantillon N°4** | Vacciné (5 mois après vaccination) | ***Positif*** |
| **Echantillon N°5** | | Négatif |
| **Echantillon N°6** | | Négatif |
| **Echantillon N°7** | | Négatif |
| **Echantillon N°8** | | ***Positif*** |
| **Echantillon N°9** | | Négatif |
| **Echantillon N°10** | | Négatif |
| **Echantillon N°11** | | Négatif |
| **Echantillon N°12** | | Négatif |
| **Echantillon N°13** | | Négatif |
| **Echantillon N°14** | | ***Positif*** |
| **Echantillon N°15** | Vacciné et naturellement infecté (6 semaines après infection) | Négatif |

Ces résultats montrent que la technologie VNT ne permet pas de détecter efficacement des anticorps de CPV dans des échantillons biologiques vaccinés depuis 5 mois (<30% de positifs dans le panel d'échantillons testés)

### 1.6.1.3. ELISA de type indirect basé sur la rB5 (comparatif):

Pour le test ELISA basé sur la rB5, le seuil de positivité est de 0,514. Les échantillons considérés comme positifs sont indiqués en caractère gras, italique et souligné.

**Tableau 3 :**

| **Echantillons** | **Description des échantillons** | **Densité optique à 450 nm** | **Statut** |
|---|---|---|---|
| **Echantillon N°1** | Infecté expérimentalement (37 jours après infection) | ***1.985*** | ***Positif*** |
| **Echantillon N°2** | Vacciné (50 jours après vaccination) et infecté expérimentalement (29 jours après infection) | ***0.680*** | ***Positif*** |
| **Echantillon N°3** | Infecté expérimentalement (29 jours après infection) | 0,194 | Négatif |
| **Echantillon N°4** | Vacciné (5 mois après vaccination) | 0,480 | Négatif |
| **Echantillon N°5** | | 0,457 | Négatif |
| **Echantillon N°6** | | ***0,857*** | ***Positif*** |
| **Echantillon N°7** | | ***0,542*** | ***Positif*** |
| **Echantillon N°8** | | 0,211 | Négatif |
| **Echantillon N°9** | | 0,211 | Négatif |
| **Echantillon N°10** | | ***0,633*** | ***Positif*** |
| **Echantillon N°11** | | 0,468 | Négatif |
| **Echantillon N°12** | | 0,357 | Négatif |
| **Echantillon N°13** | | ***0.831*** | ***Positif*** |
| **Echantillon N°14** | | 0,450 | Négatif |
| **Echantillon N°15** | Vacciné et naturellement infecté (6 semaines après infection) | 0,157 | Négatif |

Les résultats montrent que la technologie ELISA indirect avec la protéine recombinante rB5 ne permet de détecter que 36% d'échantillons positifs dans les échantillons biologiques vaccinés depuis 5 mois.

Les résultats montrent aussi que cette technologie ne permet pas de détecter 2 des 3 échantillons infectés ce qui montre une limite dans la sensibilité de la technologie.

### 1.6.1.4. ELISA de type indirect basé sur la rA33 (comparatif):

Pour le test ELISA basé sur la rA33, le seuil de positivité est de 0,277. Les échantillons considérés comme positifs sont indiqués en caractère gras, italique et souligné.

**Tableau 4 :**

| **Echantillons** | **Description des échantillons** | **Densité optique à 450 nm** | **Statut** |
|---|---|---|---|
| **Echantillon N°1** | Infecté expérimentalement (37 jours après infection) | ***3,399*** | ***Positif*** |
| **Echantillon N°2** | Vacciné (50 jours après vaccination) et infecté expérimentalement (29 jours après infection) | ***1,379*** | ***Positif*** |
| **Echantillon N°3** | Infecté expérimentalement (29 jours après infection) | ***1,147*** | ***Positif*** |
| **Echantillon N°4** | Vacciné (5 mois après vaccination) | 0,207 | Négatif |
| **Echantillon N°5** | | 0,068 | Négatif |
| **Echantillon N°6** | | ***0,299*** | ***Positif*** |
| **Echantillon N°7** | | 0,192 | Négatif |
| **Echantillon N°8** | | ***1,589*** | ***Positif*** |
| **Echantillon N°9** | | 0,092 | Négatif |
| **Echantillon N°10** | | ***0,560*** | ***Positif*** |
| **Echantillon N°11** | | 0,078 | Négatif |
| **Echantillon N°12** | | 0,229 | Négatif |
| **Echantillon N°13** | | 0,166 | Négatif |
| **Echantillon N°14** | | 0,128 | Négatif |
| **Echantillon N°15** | Vacciné et naturellement infecté (6 semaines après infection) | 0,227 | Négatif |

Ces résultats montrent que la technologie ELISA indirect avec la protéine recombinante rA33 est encore moins fiable qu'avec l'antigène rB5, puisqu'elle ne permet de détecter que 27% d'échantillons positifs dans les échantillons biologiques vaccinés depuis 5 mois.

### 1.6.1.5. ELISA de type indirect basé sur la rP32 (comparatif):

Pour le test ELISA basé sur la rP32, le seuil de positivité est de 0,282. Les échantillons considérés comme positifs sont indiqués en caractère gras, italique et souligné.

**Tableau 5**

| **Echantillons** | **Description des échantillons** | **Densité optique à 450 nm** | **Statut** |
|---|---|---|---|
| **Echantillon N°1** | Infecté expérimentalement (37 jours après infection) | ***3,099*** | ***Positif*** |
| **Echantillon N°2** | Vacciné (50 jours après vaccination) et infecté expérimentalement (29 jours après infection) | ***1,334*** | ***Positif*** |
| **Echantillon N°3** | Infecté expérimentalement (29 jours après infection) | ***0,920*** | ***Positif*** |
| **Echantillon N°4** | Vacciné (5 mois après vaccination) | ***0,290*** | ***Positif*** |
| **Echantillon N°5** | | 0,125 | Négatif |
| **Echantillon N°6** | | 0,222 | Négatif |
| **Echantillon N°7** | | ***0,287*** | ***Positif*** |
| **Echantillon N°8** | | ***0,436*** | ***Positif*** |
| **Echantillon N°9** | | 0,147 | Négatif |
| **Echantillon N°10** | | 0,270 | Négatif |
| **Echantillon N°11** | | 0,166 | Négatif |
| **Echantillon N°12** | | 0,166 | Négatif |
| **Echantillon N°13** | | 0,155 | Négatif |
| **Echantillon N°14** | | 0,120 | Négatif |
| **Echantillon** N°15 | Vacciné et naturellement infecté (6 semaines après infection) | ***0,291*** | ***Positif*** |

Ces résultats montrent que la technologie ELISA indirect avec la protéine recombinante rP32 permet de détecter que 27% d'échantillons positifs dans les échantillons biologiques vaccinés depuis 5 mois. Ce test permet la détection de l'échantillon N°15, qui n'est pas détecté par la VNT, l'IPMA, et les tests Indirect basé sur la rB5 et la rA33.

### 1.6.1.6. ELISA double antigène basé sur la rP32 (invention) :

Pour le test ELISA double antigène basé sur la rP32, le seuil de positivité utilisé est de 0,235.

**Tableau 6**

| **Echantillons** | **Description des échantillons** | **Densité optique à 450 nm** | **Statut** |
|---|---|---|---|
| **Echantillon N°1** | Infecté expérimentalement (37 jours après infection) | ***3,524*** | ***Positif*** |
| **Echantillon N°2** | Vacciné (50 jours après vaccination) et infecté expérimentalement (29 jours après infection) | ***2,214*** | ***Positif*** |
| **Echantillon N°3** | Infecté expérimentalement (29 jours après infection) | ***2,965*** | ***Positif*** |
| **Echantillon N°4** | | ***2,860*** | ***Positif*** |
| **Echantillon N°5** | | 0,195 | Négatif |
| **Echantillon N°6** | | ***1,905*** | ***Positif*** |
| **Echantillon N°7** | | ***1,498*** | ***Positif*** |
| **Echantillon N°8** | Vacciné (5 mois après vaccination) | ***2,986*** | ***Positif*** |
| **Echantillon N°9** | | 0,121 | Négatif |
| **Echantillon N°10** | | ***2,346*** | ***Positif*** |
| **Echantillon N°11** | | 0,125 | Négatif |
| **Echantillon N°12** | | ***0,954*** | ***Positif*** |
| **Echantillon N°13** | | ***0,931*** | ***Positif*** |
| **Echantillon N°14** | | ***0,657*** | ***Positif*** |
| **Echantillon N°15** | Vacciné et naturellement infecté (6 semaines après infection) | ***2,815*** | ***Positif*** |

Ces résultats montrent, de manière surprenante et inattendue, que la technologie ELISA double antigène selon l'invention basée sur la rP32 permet de détecter 73% d'échantillons positifs dans les échantillons biologiques vaccinés depuis 5 mois, ce qui est largement supérieur à l'ensemble des techniques testées.

De plus ce test permet aussi la détection de l'échantillon N°15, qui n'est pas détecté par la VNT, l'IPMA, et les tests Indirect basé sur la rB5 et la rA33. Ces deux éléments confirment que ce test utilisant la rP32 comme antigène CPV candidat et la technologie ELISA double antigène semble être plus sensible et le plus spécifique de tous les tests évalués.

En conclusion l'ensemble des résultats obtenus est résumé par le tableau 7 ci-dessous :

**Tableau 7 :**

| | Echantillons | Description des échantillons | ELISA Indire ct protéi ne B5 | ELISA Indirect protéine A33 | ELISA Indirect protéine p32 | ELISA Double antigène basé sur la p32 (invention) |
|---|---|---|---|---|---|---|
| Sensibilité | Echantillon N°1 | Infecté (37 jours après infection) | + | + | + | + |
| | Echantillon N°2 | Vacciné (50 jours après vaccination) et infecté expérimentalement (29 jours après infection) | + | + | + | + |
| | Echantillon N°3 | Infecté (29 jours après infection) | - | + | + | + |
| | Echantillon 4 à 14 | Vacciné (5 mois après vaccination) | **3+/11** | **3+/11** | **3+/11** | **8+/11** |
| | Echantillon N°15 | Vacciné puis infecté naturellement (6 semaines après infection) | - | - | + | + |
| Spécificité | 32 échantillons négatifs | Sérums de zone non endémique et non vaccinée | **27/32** | **28/32** | **29/32** | **32/32** |

De manière surprenante et inattendue, la méthode de dosage immunologique à double antigène (rP32) selon l'invention permet d'obtenir les meilleurs résultats en termes de spécificité et sensibilité comparativement aux autres techniques testées et/ou aux autres antigènes CPV candidats, avec des performances inégalées sur des échantillons biologiques vaccinés depuis plus de 5 mois, ce qui en fait une méthode de choix pour indiquer le signe d'un contact de l'animal avec un virus de la famille CPV, tel que la dermatose nodulaire contagieuse, que le contact soit issu d'une vaccination ou d'une infection naturelle.

Ces résultats ont été confirmés par des résultats obtenus sur le terrain sur des cheptels bovins à plus grande échelle, selon le protocole suivant :
- des échantillons ont été récoltés dans 2 cheptels bovins différents, vaccinés avec un vaccin commercial (MSD Lumpyvvaxs^{®} (LSDV field attenuated strain, SIS strain)
- 48 échantillons ont été prélevés 58 jours après vaccination et 48 autres échantillons ont été prélevés 87 jours après vaccination.
- ces sérums ont été testés en parallèles sur les protéines recombinantes décrites précédemment (exemple 1) en test indirect (comparatif) et selon la méthode double antigène de l'invention telle que décrite à l'exemple 1.

Les résultats sont présentés dans le tableau 8 ci-dessous :

**Tableau 8 :**

| | | ELISA Double antigène (conjugué HRP p32 recombinante décrit à l'exemple 1.1) **invention** | ELISA indirect (conjugué HRP anticorps polyclonal anti-IgG de bovin décrit à l'exemple 1.3) **comparatif** | | | ELISA Double antigène (conjugué HRP A33 recombinante décrit à l'exemple 1.2) **comparatif** |
|---|---|---|---|---|---|---|
| **Plaque sensibilisée avec protéine** | | **p32** | **p32** | **A33** | **B5** | **A33** |
| 48 sérums vaccinés 58 jours post-vaccination | Nombre de positifs | 36 | 15 | 29 | 22 | 22 |
| | % de positif | 75% | 31% | 60% | 46% | 46% |
| 48 sérums vaccinés 87 jours post-vaccination | Nombre de positifs | 30 | 13 | 32 | 20 | 27 |
| | % de positif | 63% | 27% | 67% | 42% | 56% |
| | Spécificité (n=128) | 99,70% | 100% | 96% | 86,20% | 95% |

Ces résultats confirment que :
- le test indirect (comparatif) utilisant un anticorps secondaire couplé à la péroxydase comme conjugué ne détecte que 31% des animaux vaccinés, contrairement au test utilisant la protéine P32 selon la méthode de l'invention qui permet de porter le taux de détection a 75% en maintenant la spécificité à un niveau considéré par l'homme du métier comme très élevé (>99.5%) ;
- bien que la protéine A33 semble également présenter sur le test indirect un certain potentiel diagnostique (détection de 60% à 67% des échantillons positifs) malgré une spécificité inférieure de 96% (qui peut être considérée comme trop faible pour envisager une application diagnostique commerciale), l'utilisation de cette protéine A33 selon la méthode de l'invention (c'est-à-dire à la fois comme antigène sensibilisant et comme conjugué couplé à la péroxydase) n'a pas permis de constater, contrairement à ce qui a été observé de manière originale avec la protéine P32, d'amélioration de la sensibilité.

Ces résultats confirment donc que l'effet technique sur les améliorations de la sensibilité et de la spécificité du test sont liées à cette double sélection de la protéine P32, parmi les différentes protéines disponibles, et de la méthode à double antigène, parmi les méthodes également disponibles.

### EXEMPLE 2 : Validation de l'exclusivité du test vis-à-vis du genre Capripoxvirus

### 2.1 Test de sérums issus de zone infectée par Parapox virus (Grèce) :

Les sérums de 8 animaux issus de zone infectée (Grèce) par le virus de la stomatite papuleuse bovine (Parapox) ont été testés sur l'ELISA double antigène p32 selon l'invention tel que décrit à l'exemple 1 et en parallèle par la technique de référence d'immuno-diffusion en milieu gélosé, autrement nommée technique AGID (Agar Gel ImmunoDiffusion).

Les résultats sont présentés dans le tableau 9 ci-dessous :

**Tableau 9 :**

| **Numéro d'échantillon** | **Résultats AGID (référence, comparatif)** | **Résultats ELISA double antigène p32 (invention)** |
|---|---|---|
| 1 | positif | négatif |
| 2 | positif | négatif |
| 3 | positif | négatif |
| 4 | négatif | négatif |
| 9 | faible positif | négatif |
| 13 | négatif | négatif |
| 22 | positif | négatif |
| 24 | négatif | négatif |

Parmi ces 8 sérums, 5 ont été trouvés positifs et 3 ont été trouvés négatifs par la technique AGID. En revanche, ces 8 sérums sont tous trouvés négatifs sur l'ELISA double antigène p32 Capripox virus. Ces résultats montrent que selon la méthode de l'invention, il n'y a pas de réaction croisée entre P32 de Capripox virus et une infection à Parapox virus, en faveur d'une meilleure sélectivité et exclusivité intra-espèce de la méthode de l'invention..

### 2.2 Test de sérums infectés par le virus de la stomatite papuleuse bovine (Parapox) (FLI, Allemagne) :

Les sérums de 5 animaux présentant des signes cliniques d'infection naturelle à la stomatite papuleuse bovine (Parapox) ont été prélevés à deux différents temps après l'infection (t1 and t2), espacés de 21 jours et testés parallèlement en immunofluorescence, en PCR et sur l'ELISA double antigène p32 de l'invention tel que décrit à l'exemple 1.

Les résultats sont présentés dans le tableau 10 ci-dessous :

**Tableau 10 :**

| **Test de sérums d'animaux infectés par le virus de la stomatite papuleuse bovine (parapox)** | **Résultats d'immunofluorescence (référence, comparatif)** | | **Résultats qPCR (référence, comparatif)** | **Résultats ELISA double antigène p32 (invention)** |
|---|---|---|---|---|
| **numéro d'échantillon** | **t1** | **t2** | **t2** | **t2** |
| 13 | faible positif | faible positif | positif | négatif |
| 14 | négatif | positif | positif | négatif |
| 15 | positif | positif | positif | négatif |
| 16 | négatif | positif | positif | négatif |
| 17 | négatif | positif | positif | négatif |

Les 5 cinq sérums ont été trouvés positifs en PCR et en immunofluorescence mais négatifs sur l'ELISA double antigène p32. Ces résultats montrent que selon la méthode de l'invention, il n'y a pas de réaction croisée entre P32 de Capripox virus et une infection à Parapox virus, en faveur d'une meilleure sélectivité et exclusivité intra-espèce de la méthode de l'invention comparativement aux autres méthodes disponibles.

Ces résultats permettent de démontrer que des anti-sera présentant des anticorps anti-Parapoxvirus, provenant d'animaux dont le virus isolé à partir de ces derniers a été caractérisé par PCR, et les anti-sera déterminés comme réagissant contre cette famille de virus par une technique d'immunofluorescence indirecte, ne réagissent pas sur notre test Capripox virus. Ces éléments complémentaires démontrent que la P32 est bien exclusive vis-à-vis de la famille testée, et que par extension, un test employant une P32 de la famille des Orthopox (Cowpoxvirus et Camelpoxvirus) serait spécifique de cette famille Orthopox de virus.

### EXEMPLE 3 : Exemple de protocole de kit selon l'invention :

Le kit de l'invention peut comprendre les éléments et composés suivants :
- Microplaques sensibilisées avec l'antigène purifié P32 CPV
- Conjugué contenant au moins un antigène secondaire comprenant une protéine synthétique ou recombinante rP32 ou un fragment peptidique antigénique de celle-ci et un marqueur
- Contrôle positif constitué d'un échantillon contenant des anticorps anti-CPV (OPV)
- Contrôle négatif constitué d'un échantillon biologique ne contenant pas d'anticorps anti-CPV (OPV)
- Tampon de dilutions au besoin (pour l'échantillon, le conjugué)
- Solutions de lavage, substrat (révélateur), et d'arrêt de la réaction

Selon un mode particulier, le kit de l'invention comprend :
- Microplaques de polystyrène 96 puits sensibilisées avec l'antigène purifié CPV comprenant une protéine synthétique ou recombinante rP32 ou un fragment peptidique antigénique de celle-ci ;
- Conjugué comprenant l'antigène purifié CPV comprenant une protéine synthétique ou recombinante rP32 ou un fragment peptidique antigénique de celle-ci et une peroxydase comme marqueur
- Contrôle positif constitué d'un échantillon contenant des anticorps anti-CPV (OPV)
- Contrôle négatif constitué d'un échantillon biologique ne contenant pas d'anticorps anti-CPV (OPV)
- Tampon de dilutions au besoin (pour l'échantillon, le conjugué)
- Solutions de lavage, substrat (révélateur), et d'arrêt de la réaction.

## Revendications

1. Méthode de dosage immunologique d'un anticorps spécifique d'un antigène d'un virus du genre Capripoxvirus, dans un échantillon biologique comprenant les étapes suivantes :
(i) Mise en contact d'au moins antigène primaire comprenant au moins une protéine synthétique ou recombinante rP32 ou un fragment peptidique antigénique de celle-ci, avec un échantillon biologique suspecté de contenir ledit anticorps spécifique dudit antigène primaire, dans des conditions permettant la formation d'un complexe antigène primaire / anticorps' ;
(ii) Ajout d'au moins un conjugué contenant au moins un antigène secondaire comprenant une protéine synthétique ou recombinante rP32 ou un fragment peptidique antigénique de celle-ci et un marqueur dans des conditions permettant la formation d'un complexe antigène primaire / anticorps / conjugué ; et
(iii) Détection dudit complexe antigène primaire / anticorps/ conjugué.

2. Méthode de dosage immunologique selon la revendication 1, dans laquelle l'antigène primaire et le conjugué comprennent au moins une séquence en acides aminés choisie dans le groupe constitué par :
a) la séquence SEQ ID N° 1 ou une séquence présentant au moins 90% d'identité, de préférence au moins 95% d'identité respectivement avec la SEQ ID N°1, ou
b) un fragment peptidique antigénique d'au moins 10 acides aminés de la SEQ ID N° 1, ou une séquence présentant au moins 90% d'identité, de préférence au moins 95% d'identité avec ledit fragment peptidique antigénique,
le pourcentage d'identité étant déterminé sur la base d'un alignement des séquences prises dans leur intégralité sur toute la longueur.

3. Méthode de dosage immunologique selon la revendication 1 ou 2, dans laquelle ledit échantillon biologique est choisi parmi un échantillon de sang, sérum, plasma, lait, salive, expectoration, fluide cérébrospinal, exsudats de tissus, ou fécès, de préférence un échantillon de sérum ou de plasma.

4. Méthode de dosage immunologique selon l'une des revendications 1 à 3, dans laquelle ledit échantillon biologique est un échantillon de toute espèce animale sensible à un virus du genre Capripoxvirus, en particulier un mammifère, de préférence choisi dans le groupe constitué des bovins, des ovins, et des caprins.

5. Méthode de dosage immunologique selon l'une des revendications 1 à 4, dans laquelle ledit marqueur est choisi dans le groupe constitué d'une enzyme, un composé fluorescent ou un fluorophore, un composé (chimio)luminescent, un élément radioactif, une particule d'or colloïdale ou de latex, de préférence une enzyme, de préférence encore une enzyme choisie parmi la peroxydase et la phosphatase alcaline.

6. Méthode de dosage immunologique selon l'une quelconque des revendications 1 à 5, dans laquelle ledit dosage immunologique est un dosage par immunochromatographie ou un dosage enzymo-immunologique en phase solide (ELISA), de préférence un dosage enzymo-immunologique en phase solide (ELISA).

7. Kit de détection et/ou quantification d'un anticorps spécifique d'un antigène d'un virus du genre Capripoxvirus, dans un échantillon biologique comprenant :
(i) Un support solide sensibilisé avec au moins un antigène primaire d'un virus du genre Capripoxvirus, comprenant au moins une protéine synthétique ou recombinante rP32 ou un fragment peptidique antigénique de celle-ci,
(ii) au moins un conjugué comprenant un antigène secondaire d'un virus du genre Capripoxvirus, comprenant au moins une protéine synthétique ou recombinante rP32 ou un fragment peptidique antigénique de celle-ci, et un marqueur,
(iii) Et éventuellement des moyens de développer et détecter la présence du complexe antigène primaire / anticorps/ conjugué.

8. Kit selon la revendication 7, dans lequel l'antigène primaire et le conjugué comprennent au moins une séquence en acides aminés choisie dans le groupe constitué par :
a) la séquence SEQ ID N° 1, ou une séquence présentant au moins 90% d'identité, de préférence au moins 95% d'identité respectivement avec la séquence SEQ ID N°1, ou
b) un fragment peptidique antigénique d'au moins 10 acides aminés de la séquence SEQ ID N° 1, ou une séquence présentant au moins 90% d'identité, de préférence au moins 95% d'identité avec ledit fragment peptidique antigénique, et le marqueur est une enzyme, de préférence la peroxydase. , le pourcentage d'identité étant déterminé sur la base d'un alignement des séquences prises dans leur intégralité sur toute la longueur.

9. Utilisation de la méthode de dosage immunologique selon l'une quelconque des revendications 1 à 6 ou du kit selon la revendication 7 ou 8, pour détecter et diagnostiquer *in vitro,* à partir d'un échantillon biologique, une infection causée par un virus du genre Capripoxvirus tels que le virus de la dermatose nodulaire contagieuse, le virus de la variole caprine et le virus de la clavelée, en particulier à un temps t d'au moins 2 à 3 mois post-infection.

10. Utilisation de la méthode *in vitro* de dosage immunologique selon l'une quelconque des revendications 1 à 6 ou du kit selon la revendication 7 ou 8, pour suivre dans le temps une vaccination contre un virus du genre Capripoxvirus tels que le virus de la dermatose nodulaire contagieuse, le virus de la variole caprine et le virus de la clavelée, en particulier à un temps t d'au moins 2 à 3 mois post-vaccination.

## Patentansprüche

1. Verfahren zur immunologischen Dosierung eines spezifischen Antikörpers eines Antigens eines Virus der Gattung Capripoxvirus in einer biologischen Probe, das die folgenden Schritte umfasst:
(i) Inkontaktbringen mindestens eines primären Antigens, das mindestens ein synthetisches oder rekombinantes rP32-Protein oder ein antigenes Peptidfragment davon umfasst, mit einer biologischen Probe, bei welcher der Verdacht besteht, dass sie den spezifischen Antikörper des primären Antigens enthält, unter Bedingungen, die die Bildung eines Komplexes "primäres Antigen / Antikörper" ermöglichen;
(ii) Hinzufügen mindestens eines Konjugats, das mindestens ein sekundäres Antigen, das ein synthetisches oder rekombinantes rP32-Protein oder ein antigenes Peptidfragment davon umfasst, und einen Marker enthält, unter Bedingungen, die die Bildung eines Komplexes "primäres Antigen / Antikörper / Konjugat" ermöglichen; und
(iii) Feststellung des Komplexes primäres Antigen / Antikörper / Konjugat.

2. Verfahren zur immunologischen Dosierung nach Anspruch 1, wobei das primäre Antigen und das Konjugat mindestens eine Aminosäuresequenz umfassen, die aus der Gruppe ausgewählt wird, die besteht aus:
a) der Sequenz SEQ ID NR. 1 oder einer Sequenz, die mindestens 90 % Identität, beziehungsweise vorzugsweise mindestens 95 % Identität, mit der SEQ ID NR. 1 aufweist, oder
b) einem antigenen Peptidfragment von mindestens 10 Aminosäuren der SEQ ID NR. 1 oder einer Sequenz, die mindestens 90 % Identität, vorzugsweise mindestens 95 % Identität, mit dem antigenen Peptidfragment aufweist,
wobei der Prozentsatz der Identität basierend auf einem Alignment der Sequenzen in ihrer Gesamtheit über die gesamte Länge bestimmt wird.

3. Verfahren zur immunologischen Dosierung nach Anspruch 1 oder 2, wobei die biologische Probe aus einer Blut-, Serum-, Plasma-, Milch-, Speichel-, Auswurf-, Zerebrospinalflüssigkeits-, Gewebeexsudat-, oder Fäkalienprobe, vorzugsweise einer Serum- oder Plasmaprobe, ausgewählt wird.

4. Verfahren zur immunologischen Dosierung nach Anspruch 1 bis 3, wobei die biologische Probe eine Probe von jeder Tierart ist, die gegenüber einem Virus der Gattung Capripoxvirus empfindlich ist, insbesondere von einem Säugetier, das vorzugsweise aus der Gruppe ausgewählt wird, die aus Rindern, Schafen und Ziegen besteht.

5. Verfahren zur immunologischen Dosierung nach einem der Ansprüche 1 bis 4, wobei der Marker aus der Gruppe ausgewählt wird, die aus einem Enzym, einer fluoreszierenden Verbindung oder einem Fluorophor, einer (chemi)lumineszenten Verbindung, einem radioaktiven Element, einem kolloidalen oder Latexpartikel, vorzugsweise einem Enzym, mehr zu bevorzugen einem Enzym besteht, das aus Peroxydase und alkalischer Phosphatase ausgewählt wird.

6. Verfahren zur immunologischen Dosierung nach einem der Ansprüche 1 bis 5, wobei die immunologische Dosierung eine Dosierung durch Immunchromatographie oder eine enzymimmunologische Dosierung in der Festphase (ELISA), vorzugsweise eine enzymimmunologische Dosierung in der Festphase (ELISA), ist.

7. Kit zur Feststellung und/oder Quantifizierung eines spezifischen Antikörpers eines Antigens eines Virus der Gattung Capripoxvirus in einer biologischen Probe, das umfasst:
(i) einen festen Träger, der mit mindestens einem primären Antigen eines Virus der Gattung sensibilisiert ist, das mindestens ein synthetisches oder rekombinantes rP32-Protein oder ein antigenes Peptidfragment davon umfasst,
(ii) mindestens ein Konjugat, das ein sekundäres Antigen eines Virus der Gattung Capripoxvirus umfasst, das mindestens ein synthetisches oder rekombinantes rP32-Protein oder ein antigenes Peptidfragment davon und einen Marker umfasst,
(iii) und gegebenenfalls Mittel zur Entwicklung und Feststellung des Vorhandenseins des Komplexes primäres Antigen / Antikörper / Konjugat.

8. Kit nach Anspruch 7, wobei das primäre Antigen und das Konjugat mindestens eine Aminosäuresequenz umfassen, die aus der Gruppe ausgewählt ist, die besteht aus:
a) der Sequenz SEQ ID NR. 1 oder einer Sequenz, die mindestens 90 % Identität, beziehungsweise vorzugsweise mindestens 95 %, mit der SEQ ID NR. 1 aufweist, oder
b) einem antigenen Peptidfragment von mindestens 10 Aminosäuren der Sequenz SEQ ID NR. 1 oder eine Sequenz, die mindestens 90 % Identität, vorzugsweise mindestens 95 % Identität, mit dem antigenen Peptidfragment aufweist, und der Marker ein Enzym, vorzugsweise Peroxydase, ist,
wobei der Prozentsatz der Identität basierend auf einem Alignment der Sequenzen in ihrer Gesamtheit über die gesamte Länge bestimmt wird.

9. Verwendung des Verfahrens zur immunologischen Dosierung nach einem der Ansprüche 1 bis 6 oder des Kits nach Anspruch 7 oder 8 zur Feststellung und Diagnose in vitro, ausgehend von einer biologischen Probe, einer Infektion, die durch ein Virus der Gattung Capripoxvirus, wie beispielsweise das Virus der Lumpy-skin-Krankheit, das Ziegenpockenvirus und das Virus der Pockenseuche der Schafe und Ziegen, verursacht wird, insbesondere nach einer Zeit t von mindestens 2 bis 3 Monaten nach der Infektion.

10. Verwendung des in vitro Verfahrens zur immunologischen Dosierung nach einem der Ansprüche 1 bis 6 oder des Kits nach Anspruch 7 oder 8, um eine Impfung gegen ein Virus der Gattung Capripoxvirus, wie beispielsweise das Virus der Lumpy-skin-Krankheit, das Ziegenpockenvirus und das Virus der Pockenseuche der Schafe und Ziegen, mit der Zeit, insbesondere nach einer Zeit t von mindestens 2 bis 3 Monaten nach der Impfung, zu verfolgen.

## Claims

1. Immunoassay method for an antibody specific for an antigen of a virus of the Capripoxvirus genus, in a biological sample comprising the following steps:
(i) Contacting at least one primary antigen comprising at least one synthetic or recombinant rP32 protein or an antigenic peptide fragment thereof, with a biological sample suspected of containing said antibody specific for said primary antigen, under conditions allowing the formation of a 'primary antigen/antibody' complex;
(ii) Addition of at least one conjugate containing at least one secondary antigen comprising a synthetic or recombinant rP32 protein or an antigenic peptide fragment thereof and a marker under conditions allowing the formation of a 'primary antigen/antibody/conjugate' complex; and
(iii) Detection of said primary antigen/antibody/conjugate complex.

2. Immunoassay method according to claim 1, wherein the primary antigen and the conjugate comprise at least one amino acid sequence selected from the group consisting of:
a) the sequence SEQ ID NO 1 or a sequence having at least 90% identity, preferably at least 95% identity, respectively, with SEQ ID NO 1, or
b) an antigenic peptide fragment of at least 10 amino acids of SEQ ID NO 1, or a sequence having at least 90% identity, preferably at least 95% identity, with said antigenic peptide fragment,
the percentage of identity being determined on the basis of an alignment of sequences taken in their entirety over the entire length.

3. Immunoassay method according to claim 1 or 2, wherein said biological sample is selected from a blood, serum, plasma, milk, saliva, expectoration, cerebrospinal fluid, tissue exudate or feces sample, preferably a serum or plasma sample.

4. Immunoassay method according to one of claims 1 to 3, wherein said biological sample is a sample of any animal species sensitive to a virus of the Capripoxvirus genus, in particular a mammal, preferably selected from the group consisting of cattle, sheep and goats.

5. Immunoassay method according to one of claims 1 to 4, wherein said marker is selected from the group consisting of an enzyme, a fluorescent compound or a fluorophore, a chemoluminescent compound, a radioactive element, a colloidal gold or latex particle, preferably an enzyme, more preferably an enzyme selected from peroxidase and alkaline phosphatase.

6. Immunoassay method according to any one of claims 1 to 5, wherein said immunoassay method is assay by immunochromatography or a solid phase enzyme-linked immunosorbent assay (ELISA), preferably a solid phase enzyme-linked immunosorbent assay (ELISA).

7. Kit for detecting and/or quantifying an antibody specific for an antigen of a virus of the Capripoxvirus genus in a biological sample comprising:
(i) A solid support sensitized with at least one primary antigen of a virus of the Capripoxvirus genus, comprising at least one synthetic or recombinant rP32 protein or an antigenic peptide fragment thereof,
(ii) At least one conjugate comprising one secondary antigen of a virus of the Capripoxvirus genus, comprising at least one synthetic or recombinant rP32 protein or an antigenic peptide fragment thereof and a marker,
(iii) And, optionally, means for developing and detecting the presence of the primary antigen/antibody/conjugate complex.

8. Kit according to claim 7, wherein the primary antigen and the conjugate comprise at least one amino acid sequence selected from the group consisting of:
a) the sequence SEQ ID NO 1 or a sequence having at least 90% identity, preferably at least 95% identity, respectively, with sequence SEQ ID NO 1, or
b) an antigenic peptide fragment of at least 10 amino acids of the sequence SEQ ID NO 1, or a sequence having at least 90% identity, preferably at least 95% identity, with said antigenic peptide fragment, and the marker is an enzyme, preferably peroxidase,
the percentage of identity being determined on the basis of an alignment of sequences taken in their entirety over the entire length.

9. Use of the immunoassay method according to any one of claims 1 to 6 or the kit according to claim 7 or 8, to detect and diagnose *in vitro,* from a biological sample, an infection caused by a virus of the Capripoxvirus genus such as lumpy skin disease virus, goat pox virus and sheep pox virus, in particular at a time t of at least 2 to 3 months post-infection.

10. Use of the *in vitro* immunoassay method according to any one of claims 1 to 6 or the kit according to claim 7 or 8, to follow over time a vaccination against a virus of the Capripoxvirus genus such as lumpy skin disease virus, goat pox virus and sheep pox virus, in particular at a time t of at least 2 to 3 months post-vaccination.


### SEQUENCE LISTING

<110> ID VET
<120> Méthode de dosage immunologique d'anticorps spécifiques d'antigènes de virus de la famille des Poxviridae
<130> B373848PCT/D36873
<150> FR 1751266
   <151> 2017-02-16
<160> 5
<170> PatentIn version 3.5
<210> 1
   <211> 322
   <212> PRT
   <213> Capripoxvirus
<220>
   <221> misc_feature
   <223> ProteIn P32 LSDV
<400> 1
   Met Ala Asp Ile Pro Leu Tyr Val Ile Pro Ile Val Gly Arg Glu Ile
   1               5                   10                  15
   Ser Asp Val Val Pro Glu Leu Lys Ser Asp Asn Asp Ile Phe Tyr Lys
               20                  25                  30
   Lys Val Asp Thr Val Lys Asp Phe Lys Asn Ser Asp Val Asn Phe Phe
           35                  40                  45
   Phe Lys Asp Lys Lys Asp Ile Ser Leu Ser Tyr Lys Phe Leu Ile Trp
       50                  55                  60
   Glu Lys Val Glu Lys Ser Gly Gly Val Glu Asn Phe Thr Glu Tyr Phe
   65                  70                  75                  80
   Ser Gly Leu Cys Asn Ala Leu Cys Thr Lys Glu Ala Lys Ser Ser Ile
                   85                  90                  95
   Val Lys His Phe Ser Leu Trp Lys Ser Tyr Ala Asp Ala Asp Ile Lys
               100                 105                 110
   Asn Ser Glu Asn Lys Phe Ile Val Val Ile Glu Asp Asp Asn Thr Leu
           115                 120                 125
   Lys Asp Leu Ile Thr Ile His Asn Ile Ile Ile Glu Met Gln Glu Lys
       130                 135                 140
   Asn Ile Asp Ile Phe Gln Leu Arg Glu Thr Phe His Asn Ser Asn Ser
   145 150 155 160
   Arg Ile Leu Phe Asn Gln Glu Asn Asn Asn Phe Met Tyr Ser Tyr Thr
   165 170 175
   Gly Gly Tyr Asp Phe Thr Leu Ser Ala Tyr Val Ile Arg Leu Ser Ser
   180 185 190
   Ala Ile Lys Ile Ile Asn Glu Ile Ile Lys Asn Lys Gly Ile Ser Thr
   195 200 205
   Ser Leu Ser Phe Glu Met Tyr Lys Leu Glu Lys Glu Leu Lys Leu Asn
   210 215 220
   Arg Gln Val Leu Asn Asp Ser Ser Lys Tyr Ile Leu His Asn Thr Lys
   225 230 235 240
   Tyr Leu Ser Lys Lys Arg Ala Asn Glu Met Lys Asn Gly Ile Trp Asn
   245 250 255
   Arg Val Gly Lys Trp Met Ala His Arg Phe Pro Asp Phe Ser Tyr Tyr
   260 265 270
   Ile Ser His Pro Leu Val Ser Phe Phe Gly Ile Phe Asp Ile Ser Ile
   275 280 285
   Ile Gly Ala Leu Ile Ile Leu Phe Ile Ile Ile Met Ile Ile Phe Asp
   290 295 300
   Leu Asn Ser Lys Leu Leu Trp Phe Leu Ala Gly Met Leu Phe Thr Tyr
   305 310 315 320
   Ile Ile
<210> 2
   <211> 196
   <212> PRT
   <213> Capripoxvirus
<220>
   <221> misc_feature
   <223> Protéine A33
<400> 2
   Met Leu Val Asp Ile Pro Lys Ser Gly Thr Glu Thr Asp Tyr Asp Glu
   1 5 10 15
   Ser Asn Asn Phe Thr Ala Phe Ala Gly Ser Thr Ile Tyr Gly Tyr Gly
   20 25 30
   Leu Lys Ser Lys Lys Asn Ile Lys Lys Lys Val Lys Leu Ile Asn Phe
   35 40 45
   Cys Ile Lys Ile Ser Ile Met Ala Ser Met Val Ser Leu Ile Thr Ile
   50 55 60
   Thr Ile Leu Leu Ala Phe Phe Asn Asn Thr Cys Glu Leu Asn Gln Phe
   65 70 75 80
   Lys Glu His Lys Pro Tyr Phe Leu Lys Asn Pro Asn Pro Thr Thr Tyr
   85 90 95
   Ser Asp Asp Asp Thr Glu Ser Glu Leu Asn Val Tyr Arg Ser Cys Lys
   100 105 110
   Gly Ile Val Tyr Ser Gly Tyr Cys Tyr Thr Phe Asn Ser Glu Pro Lys
   115 120 125
   Ser Phe Asn Asp Ala Tyr Asp Asp Cys Glu Lys Lys Asn Ser Glu Leu
   130 135 140
   Pro Ser Asn Asn Leu Met Asn Asp Trp Ile Ser Asp Tyr Leu Asp Gly
   145 150 155 160
   Thr Trp Gly Glu Asp Gly Asn Val Leu Phe Lys Glu Lys Asn Gln Glu
   165 170 175
   Leu Glu Thr Ile Asp Ile Ser Asp Glu Met Arg Ser Tyr Tyr Cys Val
   180 185 190
   Arg Ser Phe Phe
   195
<210> 3
   <211> 225
   <212> PRT
   <213> Capripoxvirus
<220>
   <221> misc_feature
   <223> Protéine B5
<400> 3
   Met Lys Asn Ile His Met Leu Leu Ile Leu Leu Cys Asn Lys Val Tyr
   1               5                   10                  15
   Ser Leu Cys Asp Leu Asn Lys Cys Cys Tyr Pro Pro Ser Ile Lys Asn
               20                  25                  30
   Gly Tyr Ile Tyr Asn Glu Lys Thr Glu Tyr Asn Ile Gly Ser Asn Val
           35                  40                  45
   Thr Phe Phe Cys Gly Asn Asn Thr Arg Gly Val Ser Tyr Thr Leu Val
       50                  55                  60
   Gly Glu Lys Asn Ile Ile Cys Glu Lys Asp Gly Lys Trp Asn Lys Glu
   65                  70                  75                  80
   Phe Pro Val Cys Lys Ile Ile Arg Cys Arg Phe Pro Ala Leu Gln Asn
                   85                  90                  95
   Gly Phe Val Asn Gly Ile Pro Asp Ser Arg Lys Phe Tyr Tyr Glu Ser
               100                 105                 110
   Glu Val Ser Phe Ser Cys Lys Pro Gly Phe Val Leu Ile Gly Thr Lys
           115                 120                 125
   Tyr Ser Val Cys Gly Ile Asn Ser Ser Trp Ile Pro Lys Val Pro Ile
       130                 135                 140
   Cys Ser Arg Asp Asn Ile Thr Tyr Asn Lys Ile Tyr Ile Asn Lys Val
   145                 150                 155                 160
   Asn Ile Asp Asp Asn Phe Phe Asn Gln Ile Asn Asn Ser Asn Thr Tyr
                   165                 170                 175
   Tyr Phe Asp Lys Ile Leu Gln Ile Asn Asn Val Asn Arg Tyr Thr Leu
               180                 185                 190
   Ile Phe Phe Val Val Val Ser Ile Lys Ile Leu Phe Gly Phe Val Phe
           195                 200                 205
   Ile Phe Phe Ser Cys Asn Lys Lys Pro Leu Asp Ser Ile Lys Tyr Tyr
       210                 215                 220
   Lys
   225
<210> 4
   <211> 324
   <212> **PRT**
   <213> Cowpoxvirus
<220>
   <221> misc_feature
   <223> Protéine H3L CWPV
<400> 4
   Met Ala Ala Val Lys Thr Pro Val Ile Val Val Pro Val Ile Asp Arg
   1               5                   10                  15
   Pro Pro Ser Glu Thr Phe Pro Asn Val His Glu His Ile Asn Asp Gln
               20                  25                  30
   Lys Phe Asp Asp Val Lys Asp Asn Glu Val Met Pro Glu Lys Arg Asn
           35                  40                  45
   Val Val Val Val Lys Asp Asp Pro Asp His Tyr Lys Asp Tyr Ala Phe
       50                  55                  60
   Ile Gln Trp Thr Gly Gly Asn Ile Arg Asn Asp Asp Lys Tyr Thr His
   65                  70                  75                  80
   Phe Phe Ser Gly Phe Cys Asn Thr Met Cys Thr Glu Glu Thr Lys Arg
                   85                  90                  95
   Asn Ile Ala Arg His Leu Ala Leu Trp Asp Ser Asn Phe Phe Thr Glu
               100                 105                 110
   Leu Glu Asn Lys Lys Val Glu Tyr Val Val Ile Val Glu Asn Asp Asn
           115                 120                 125
   Val Ile Glu Asp Ile Thr Phe Leu Arg Pro Val Leu Lys Ala Met His
       130                 135                 140
   Asp Lys Lys Ile Asp Ile Leu Gln Met Arg Glu Ile Ile Thr Gly Asn
   145                 150                 155                 160
   Lys Val Lys Thr Glu Leu Val Met Asp Lys Asn His Ala Ile Phe Thr
                   165                 170                 175
   Tyr Thr Gly Gly Tyr Asp Val Ser Leu Ser Ala Tyr Ile Ile Arg Val
               180                 185                 190
   Thr Thr Ala Leu Asn Ile Val Asp Glu Ile Ile Lys Ser Gly Gly Leu
           195                 200                 205
   Ser Ser Gly Phe Tyr Phe Glu Ile Ala Arg Ile Glu Asn Glu Met Lys
       210                 215                 220
   Ile Asn Arg Gln Ile Leu Asp Asn Ala Ala Lys Tyr Val Glu His Asp
   225 230 235 240
   Pro Arg Leu Val Ala Glu His Arg Phe Glu Asn Met Lys Pro Asn Phe
   245 250 255
   Trp Ser Arg Ile Gly Thr Ala Ala Ala Lys Arg Tyr Pro Gly Val Met
   260 265 270
   Tyr Ala Phe Thr Thr Pro Leu Ile Ser Phe Phe Gly Leu Phe Asp Ile
   275 280 285
   Asn Val Ile Gly Leu Ile Val Ile Leu Phe Ile Met Phe Met Leu Ile
   290 295 300
   Phe Asn Val Lys Ser Lys Leu Leu Trp Phe Leu Thr Gly Thr Phe Val
   305 310 315 320
   Thr Ala Phe Ile
<210> 5
   <211> 324
   <212> PRT
   <213> Camelpoxvirus
<220>
   <221> misc_feature
   <223> Protéine H3L CMPV
<400> 5
   Met Ala Ala Val Lys Thr Pro Val Ile Val Val Pro Val Ile Asp Arg
   1 5 10 15
   His Pro Ser Glu Thr Phe Pro Asn Val His Glu His Ile Asn Asp Gln
   20 25 30
   Lys Phe Asp Asp Val Lys Asp Asn Glu Val Met Pro Glu Lys Arg Asp
   35 40 45
   Val Val Ile Val Lys Asp Asp Pro Asp His Tyr Lys Asp Tyr Ala Phe
   50 55 60
   Ile Gln Trp Thr Gly Gly Asn Ile Arg Asn Asp Asp Lys Tyr Thr His
   65 70 75 80
   Phe Phe Ser Gly Phe Cys Asn Thr Met Cys Thr Glu Glu Thr Lys Arg
   85 90 95
   Asn Ile Ala Arg His Leu Ala Leu Trp Asp Ser Lys Phe Phe Thr Glu
               100                 105                 110
   Leu Glu Asn Lys Lys Val Glu Tyr Val Val Ile Val Glu Asn Asp Asn
           115                 120                 125
   Val Ile Glu Asp Ile Thr Phe Leu Arg Pro Val Leu Lys Ala Met His
       130                 135                 140
   Asp Lys Lys Ile Asp Ile Leu Gln Met Arg Glu Ile Ile Thr Gly Asn
   145                 150                 155                 160
   Lys Val Lys Thr Glu Leu Val Met Asp Lys Asn Tyr Ala Ile Phe Thr
                   165                 170                 175
   Tyr Thr Gly Gly Tyr Asp Val Ser Leu Ser Ala Tyr Ile Ile Arg Val
               180                 185                 190
   Thr Thr Ala Leu Asn Ile Val Asp Glu Ile Ile Lys Ser Gly Gly Leu
           195                 200                 205
   Ser Ser Gly Phe Tyr Phe Glu Ile Ala Arg Ile Glu Asn Glu Met Lys
       210                 215                 220
   Ile Asn Arg Gln Ile Met Asp Asn Ser Ala Lys Tyr Val Glu His Asp
   225                 230                 235                 240
   Pro Arg Leu Val Ala Glu His Arg Phe Glu Asn Met Lys Pro Asn Phe
                   245                 250                 255
   Trp Ser Arg Ile Gly Thr Ala Ala Ala Lys Arg Tyr Pro Gly Val Met
               260                 265                 270
   Tyr Ala Phe Thr Thr Pro Leu Ile Ser Phe Phe Gly Leu Phe Asp Ile
           275                 280                 285
   Asn Val Ile Gly Leu Ile Val Ile Leu Phe Ile Met Phe Met Leu Ile
       290                 295                 300
   Phe Asn Val Lys Ser Lys Leu Leu Trp Phe Leu Thr Gly Thr Phe Val
   305                 310                 315                 320
   Thr Ala Phe Ile